# EUROPÄISCHE PATENTSCHRIFT

(11) **EP 0 825 178 B1**
(45) Veröffentlichungstag und Bekanntmachung des Hinweises auf die Patenterteilung: **20.06.2001**
(21) Anmeldenummer: 97114244.3
(22) Anmeldetag: 18.08.1997
(51) Int. Cl.: C07C 279/22, C07C 277/08, A61K 31/155

(54) **Phenylsubstituierte Alkenylcarbonsäure-guanidide, Verfahren zu ihrer Herstellung, ihre Verwendung als Medikament oder Diagnostikum sowie sie enthaltendes Medikament**
Phenyl-substituted alkenoylguanidides, process for their preparation, their use as drugs or in diagnostic agents and drugs containing them
Alkènoylguanidides substituées par un groupe phényle, procédé pour leur préparation, leur emploi comme médicaments ou agent de diagnostic ainsi que médicaments les contenants

(30) Priorität: 22.08.1996 DE 19633966
(43) Veröffentlichungstag der Anmeldung: 25.02.1998
(73) Patentinhaber: HOECHST AKTIENGESELLSCHAFT, 65929 Frankfurt am Main (DE)
(72) Erfinder: Schwark, Jan-Robert, Dr., 65929 Frankfurt (DE); Brendel, Joachim, Dr., 61118 Bad Vilbel (DE); Kleemann, Heinz-Werner, 65474 Bischofsheim (DE); Lang, Hans Jochen, Dr., 65719 Hofheim (DE); Weichert, Andreas, Dr., 63329 Egelsbach (DE); Jansen, Hans-Willi, Dr., 65527 Niedernhausen (DE); Scholz, Wolfgang, Dr., 65760 Eschborn (DE)

(56) Entgegenhaltungen:
- EP-A- 0 640 587
- DE-A- 4 421 536
- US-A- 4 544 670

## Beschreibung

Die Erfindung betrifft phenylsubstituierte Alkenylcarbonsäure-guanidide der Formel I 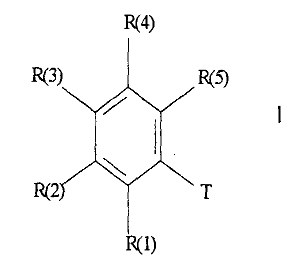 worin bedeuten:
- T: 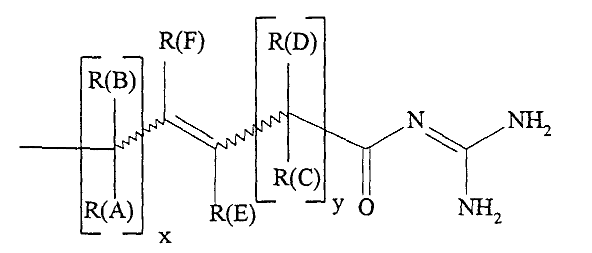 R(A) Wasserstoff, F, Cl, Br, I, CN, OH, OR(6), (C₁-C₄-Alkyl, Oᵣ(CH₂)ₐC_{b}F_{2b+1}, (C₃-C₈)-Cycloalkyl oder NR(7)R(8)
r Null oder 1;
a Null, 1, 2, 3 oder 4;
b 1, 2, 3 oder 4;
R(6) (C₁-C₄)-Alkyl, (C₁-C₄)-Perfluoralkyl, (C₃-C₆)-Alkenyl, (C₃-C₈)-Cycloalkyl, Phenyl oder Benzyl,
wobei der Phenylkern nicht substituiert oder substituiert ist mit 1 - 3 Substituenten ausgewählt aus der Gruppe bestehend aus F, Cl, CF₃, Methyl, Methoxy und NR(9)R(10);
R(9) und R(10) H, (C₁-C₄)-Alkyl oder (C₁-C₄)-Perfluoralkyl;
R(7) und R(8) unabhängig voneinander wie R(6) definiert;
oder
R(7) und R(8) gemeinsam 4 oder 5 Methylengruppen, von denen eine CH₂-Gruppe durch Sauerstoff, Schwefel, NH, N-CH₃ oder N-Benzyl ersetzt sein kann;
R(B), R(C) und R(D) unabhängig wie R(A) definiert;
x Null, 1 oder 2;
y Null, 1 oder 2;
R(F) Wasserstoff, F, Cl, Br, I, CN, OR(12), (C₁-C₈)-Alkyl, Oₚ(CH₂)_{f}C_{g}F_{2g+1}, (C₃-C₈)-Cycloalkyl oder (C₁-C₉)-Heteroaryl;
p Null oder 1;
f Null, 1, 2, 3 oder 4;
g 1, 2, 3, 4, 5, 6, 7 oder 8;
R(12) (C₁-C₈)-Alkyl, (C₁-C₄)-Perfluoralkyl, (C₃-C₈)-Alkenyl, (C₃-C₈)-Cycloalkyl, Phenyl oder Benzyl,
wobei der Phenylkern nicht substituiert ist oder substituiert mit 1 - 3 Substituenten ausgewählt aus der Gruppe bestehend aus F, Cl, CF₃, Methyl, Methoxy und NR(13)R(14);
R(13) und R(14) H, (C₁-C₄)-Alkyl oder (C₁-C₄)-Perfluoralkyl;
R(E) unabhängig wie R(F) definiert;

- R(1): unabhängig wie T definiert;
oder
- R(1): Wasserstoff, -OₖCₘH₂ₘ₊₁, -Oₙ(CH₂)ₚC_{q}F_{2q+1}, F, Cl, Br, I, CN, -(C=O)-N=C(NH₂)₂, -SOᵣR(17), -SOᵣ₂NR(31)R(32), Oᵤ(CH₂)ᵥC₆H₅ -Oᵤ₂-(C₁-C₉)-Heteroaryl oder -Sᵤ₂-(C₁-C₉)-Heteroaryl;
k Null oder 1;
m Null, 1, 2, 3, 4, 5, 6, 7 oder 8;
n Null oder 1;
p Null, 1, 2, 3 oder 4;
q 1, 2, 3, 4, 5, 6, 7 oder 8;
r Null, 1, 2;
r2 Null, 1 oder 2;
R(31) und R(32) unabhängig voneinander Wasserstoff, (C₁-C₈)-Alkyl oder (C₁-C₈)- Perfluoralkyl; oder
R(31) und R(32) gemeinsam 4 oder 5 Methylengruppen, von denen eine CH₂-Gruppe durch Sauerstoff, S, NH, N-CH ₃ oder N-Benzyl ersetzt sein kann;
R(17) (C₁-C₈)-Alkyl;
u Null oder 1;
u2 Null oder 1;
v Null oder 1, 2, 3 oder 4;
R(2), R(3), R(4) und R(5) unabhängig voneinander wie R(1) definiert,
wobei der Phenylkern unsubstituiert ist oder substituiert mit 1 - 3 Substituienten ausgewählt aus der Gruppe bestehend F, Cl, CF₃, Methyl, Methoxy, -(CH₂)_{w}NR(21)R(22), NR(18)R(19) und C₁-(C₉)- Heteroaryl;
R(18), R(19), R(21) und R(22) unabhÄngig voneinander (C₁-C₄)-Alkyl oder (C₁-C₄)- Perfluoralkyl;
w 1, 2, 3 oder 4;
wobei der Heterocyclus des (C1-C9)-Heteroaryl unsubstituiert oder substituiert ist mit 1 - 3 Substituenten ausgewählt aus der Gruppe bestehend aus F, Cl, CF₃, Methyl oder Methoxy;
R(2), R(3), R(4) und R(5) unabhängig voneinander wie R(1) definiert,
oder
- R(1) und R(2) oder R(2) und R(3): jeweils gemeinsam -CH-CH=CH-CH-,
das nicht substituiert oder substituiert ist mit 1 - 3 Substituenten ausgewählt aus der Gruppe bestehend aus F, Cl, CF₃, Methyl, Methoxy, -(CH₂)_{w2}NR(24)R(25) und NR(26)R(27);
R(24), R(25), R(26) und R(27) H, (C₁-C₄)-Alkyl oder (C₁-C₄)-Perfluoralkyl;
w2 1, 2, 3 oder 4;
wobei der Rest T im Molekül mindestens zweimal, jedoch nur höchstens dreimal vorhanden ist;
sowie deren pharmazeutisch verträgliche Salze.

Bevorzugt sind Verbindungen der Formel I, in der bedeuten:
- T: 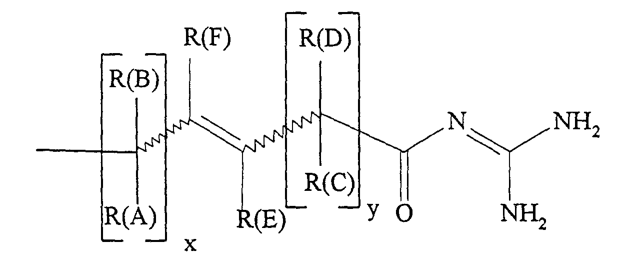 R(A) Wasserstoff, F, Cl, CN, OH, OR(6), (C₁-C₄)-Alkyl, Oᵣ(CH₂)ₐC_{b}F_{2b+1}, (C₃-C₈)-Cycloalkyl oder NR(7)R(8)
r Null oder 1;
a Null, 1 oder 2;
b 1, 2, 3 oder 4;
R(6) (C₁-C₄)-Alkyl, (C₁-C₄)-Perfluoralkyl, Phenyl oder Benzyl, wobei der Phenylkern nicht substituiert ist oder substituiert mit 1 - 3 Substituenten ausgewählt aus der Gruppe bestehend aus F, Cl, CF₃, Methyl, Methoxy und NR(9)R(10);
R(9) und R(10) unabhängig voneinander H, CH₃ oder CF₃;
R(7) und R(8) unabhängig voneinander wie R(6) definiert;
oder
R(7) und R(8) gemeinsam 4 oder 5 Methylengruppen, von denen eine CH₂-Gruppe durch Sauerstoff, Schwefel, NH, N-CH₃ oder N-Benzyl ersetzt sein kann;
R(B), R(C), R(D) unabhängig wie R(A) definiert;
x Null oder 1;
y Null oder 1;
R(F) Wasserstoff, F, Cl, CN, OR(12), (C₁-C₄)-Alkyl, Oₚ(CH₂)_{f}C_{g}F_{2g+1}, (C₃-C₈)-Cycloalkyl oder (C₁-C₉)-Heteroaryl;
p Null oder 1;
f Null, 1 oder 2;
g 1, 2, 3 oder 4;
R(12) (C₁-C₄)-Alkyl, (C₁-C₄)-Perfluoralkyl, (C₃-C₈)-Cycloalkyl, Phenyl oder Benzyl,
wobei der Phenylkern unsubstituiert ist oder substituiert ist mit 1 - 3 Substituenten ausgewählt aus der Gruppe bestehend aus F, Cl, CF₃, Methyl, Methoxy und NR(13)R(14);
R(13) und R(14) unabhängig voneinander H, CH₃ oder CF₃;
R(E) unabhängig wie R(F) definiert;
- R(1): unabhängig wie T definiert;
oder
- R(1): Wasserstoff, -OₖCₘH₂ₘ₊₁, OₙC_{q}F_{2q+1}, F, Cl, Br, I, CN, -(C=O-N=C(NH₂)₂, -SOᵣR(17), -SOᵣ₂NR(31)R(32), -Oᵤ(CH₂)ᵥC₆H₅, -Oᵤ₂-(C₁-C₉)-Heteroaryl oder Sᵤ₂-(C₁-C₉)-Heteroaryl;
k Null oder 1;
m Null, 1, 2, 3 oder 4;
n Null oder 1;
q 1, 2, 3 oder 4;
r 2;
r2 Null, 1, 2;
R(31) und R(32) unabhängig voneinander Wasserstoff, (C₁-C₄)-Alkyl oder (C₁-C₄)-Perfluoralkyl;
oder
R(31) und R(32) gemeinsam 4 oder 5 Methylengruppen, von denen eine CH₂-Gruppe durch Sauerstoff, S, NH, N-CH₃ oder N-Benzyl ersetzt sein kann;
R(17) (C₁-C₄)-Alkyl;
u Null oder 1;
u2 Null oder 1;
v Null, 1 oder 2;
wobei der Phenylkern nicht substituiert oder substituiert ist mit 1-3 Substituenten ausgewählt aus der Gruppe bestehend aus F, Cl, CF₃, Methyl, Methoxy, -(CH₂)_{w}N(21)R(22), NR(18)R(19) oder (C₁-C₉)-Heteroaryl;
R(18), R(19), R(21) und R(22) H, (C₁-C₄)-Alkyl oder (C₁-C₄)-Perfluoralkyl;
w 1, 2, 3, 4; wobei der Heterocyclus des (C₁-C₉)-Heteroaryl unsubstituiert oder substituiert ist mit 1 - 3 Substituenten ausgewählt aus der Gruppe bestehend aus F, Cl, CF₃, Methyl oder Methoxy;
- R(2), R(3), R(4) und R(5): unabhängig voneinander wie R(1) definiert,
oder
- R(1) und R(2) oder R(2) und R(3): jeweils gemeinsam -CH-CH=CH-CH-,
das nicht substituiert oder substituiert ist mit 1 - 3 Substituenten ausgewählt aus der Gruppe bestehend aus F, Cl, CF₃, Methyl, Methoxy, (CH₂)_{w2}NR(24)R(25) und NR(26)R(27);
R(24), R(25), R(26) und R(27) H, CH₃ oder CF₃;
w2 1, 2, 3 oder 4; wobei der Rest T im Molekül jedoch nur zweimal vorhanden ist; sowie deren pharmazeutisch verträgliche Salze.

Besonders bevorzugt sind Verbindungen der Formel I, in der bedeuten:
- T: 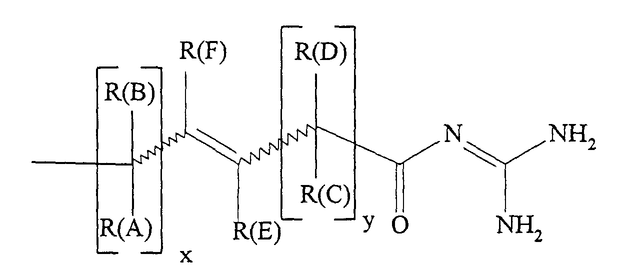 x Null;
y Null;
R(F) Wasserstoff, F, Cl, CN, OR(12), (C₁-C₄)-Alkyl, OₚC_{g}F2_{g+1}, (C₃- C₈)-Cycloalkyl oder (C₁-C₉)-Heteroaryl;
p Null oder 1;
g 1, 2, 3 oder 4;
R(12) (C₁-C₄)-Alkyl, CF₃, (C₃-C₈)-Cycloalkyl, Phenyl oder Benzyl,
wobei der Phenylkern nicht substituiert ist oder substituiert ist mit 1 - 3 Substituenten ausgewählt aus der Gruppe bestehend aus F, Cl, CF₃, Methyl oder Methoxy;
R(13) und R(14) H, CH₃ oder CF₃;
R(E) unabhängig wie R(F) definiert
- R(1): unabhängig wie T definiert,
oder
- R(1): Wasserstoff, -OₖCₘH₂ₘ₊₁, -OₙC_{q}F_{2q+1}, F, Cl, CN, -(C=O)-N=C(NH₂)₂, -SO₂CH₃, -SO₂NR(31)R(32), -Oᵤ(CH₂)ᵥC₆H₅, -Oᵤ₂-(C₁-C₉) -Heteroaryl oder Sᵤ₂-(C₁-C₉)-Heteroaryl;
k Null oder 1;
m Null, 1, 2, 3 oder 4;
n Null oder 1;
q 1, 2, 3 oder 4;
R(31) und R(32) unabhängig voneinander Wasserstoff oder (C₁-C₄)-Alkyl;
oder
R(31) und R(32) gemeinsam 4 oder 5 Methylengruppen, von denen eine CH₂-Gruppe durch Sauerstoff, S, NH, N-CH₃ oder N-Benzyl ersetzt sein kann;
u Null oder 1;
u2 Null oder 1;
v Null oder 1;
wobei der Phenylkern nicht substituiert ist oder substituiert mit 1-3 Substituenten ausgewählt aus der Gruppe bestehend aus F, Cl, CF₃, Methyl, Methoxy, -(CH₂)_{w}N(21)R(22) und NR(18)R(19);
R(18), R(19), R(21) und R(22) unabhängig voneinander H, (C₁-C₄)-Alkyl oder (C₁-C₄)-Perfluoralkyl;
w 1, 2, 3 oder 4;
wobei der Heterocyclus des (C₁-C₉)-Heteroaryl unsubstituiert oder substituiert ist mit 1 - 3 Substituenten ausgewählt aus der Gruppe bestehend aus F, Cl, CF₃, Methyl oder Methoxy;
- R(2), R(3), R(4) und R(5): unabhängig voneinander wie R(1) definiert;
oder
- R(1) und R(2) oder R(2) und R(3): jeweils gemeinsam -CH-CH=CH-CH-,
das nicht substituiert ist oder substituiert mit 1 - 3 Substituenten ausgewählt aus der Gruppe bestehend aus F, Cl, CF₃, Methyl, Methoxy, -(CH₂)_{w2}NR(24)R(25) und NR(26)R(27);
R(24), R(25), R(26) und R(27) unabhängig voneinander H, (C₁-C₄)-Alkyl oder (C₁-C₄)-Perfluoralkyl;
w2 1, 2, 3 oder 4;
wobei der Rest T im Molekül jedoch nur zweimal vorhanden ist; sowie deren pharmazeutisch verträgliche Salze.

Ganz besonders bevorzugt sind die folgenden Verbindungen:
1,2-Bis-[3-(E-2-methyl-propensäureguanidid)]benzol-dihydrochlorid,
1,3-Bis-[3-(E-2-methyl-propensäureguanidid)]benzol-dihydrochlorid,
1,4-Bis-[3-(E-2-methyl-propensäureguanidid)]benzol-dihydrochlorid,
2,3-Bis-[3-(E-2-methyl-propensäureguanidid)]naphthalin-dihydrochlorid,
1,2-Bis-[3-(Z-2-fluor-propensäureguanidid)]benzol-dihydrochlorid,
1-[3-(Z-2-fluor-propensäureguanidid)]-2-[3-(E-2-methyl-propensäureguanidid)]benzol-dihydrochlorid,
1,3-Bis-[3-(Z-2-fluor-propensäureguanidid)]benzol-dihydrochlorid,
3-(4-Chlor-3-guanidinocarbonyl-5-phenyl)phenyl-2-methyl-propensäureguanidid,
1,3-Bis-[3-(E-2-methyl-propensäureguanidid)]-2-methoxy-5-methyl-benzol-hydrochlorid,
1,2-Bis-[3-(E-2-methyl-propensäureguanidid)]-4-methyl-benzol-dihydrochlorid,
1,2-Bis-[3-(E-2-methyl-propensäureguanidid)]-4,5-dichlor-benzol-dihydrochlorid,
1,3-Bis-[3-(E-propensäureguanidid)]benzol-dihydrochlorid,
1,2-Bis-[3-(E-2-methyl-propensäureguanidid)]-4-brom-benzol-dihydrochlorid,
1,2-Bis-[3-(E-2-methyl-propensäureguanidid)]-4-(4-methoxyphenoxy)-benzol-dihydrochlorid,
1,2-Bis-[3-(E-2-methyl-propensäureguanidid)]-4-(4-methyl-phenoxy)-benzol-dihydrochlorid,
1,3-Bis-[3-(E-2-methyl-propensäureguanidid)]-5-methoxy-benzol-hydrochlorid,
1,3-Bis-[3-(E-2-methyl-propensäureguanidid)]-5-tert.-butyl-benzol-hydrochlorid,
1,4-Bis-[3-(E-2-methyl-propensäureguanidid)]-2,5-dichlor-benzol-dihydrochlorid,
1,2-Bis-[3-(E-2-methyl-propensäureguanidid)]-4-(phenoxy)-benzol-dihydrochlorid,
1,2-Bis-[3-(E-2-methyl-propensäureguanidid)]-4-(methoxy)-benzol-dihydrochlorid,
1,2-Bis-[3-(E-2-methyl-propensäureguanidid)]-4-(ethoxy)-benzol-dihydrochlorid und
1,2-Bis-[3-(E-2-methyl-propensäureguanidid)]-4-(3-pyridyloxy)-benzol-dihydrochlorid.

Enthält die Verbindungen der Formel I ein oder mehrere Asymmetriezentren, so können diese sowohl S als auch R konfiguriert sein. Die Verbindungen können als optische Isomere, als Diastereomere, als Racemate oder als Gemische derselben vorliegen.

Die Doppelbindungsgeometrie der Verbindungen der Formel I kann sowohl E als auch Z sein. Die Verbindungen können als Doppelbindungsisomere im Gemisch vorliegen.

Die bezeichneten Alkyl- und Perfluoralkyl-Reste können sowohl geradkettig wie verzweigt vorliegen.

Unter (C₁-C₉)-Heteroaryl werden insbesondere Reste verstanden, die sich von Phenyl oder Naphthyl ableiten, in welchen eine oder mehrere CH-Gruppen durch N ersetzt sind und/oder in welchen mindestens zwei benachbarte CH-Gruppen (unter Bildung eines fünfgliedrigen aromatischen Rings) durch S, NH oder O ersetzt sind. Des weiteren können auch ein oder beide Atome der Kondensationsstelle bicyclischer Reste (wie im Indolizinyl) N-Atome sein.

Als Heteroaryl gelten insbesondere Furanyl, Thienyl, Pyrrolyl, Imidazolyl, Pyrazolyl, Triazolyl, Tetrazolyl, Oxazolyl, Isoxazolyl, Thiazolyl, Isothiazolyl, Pyridyl, Pyrazinyl, Pyrimidinyl, Pyridazinyl, Indolyl, Indazolyl, Chinolyl, Isochinolyl, Phthalazinyl, Chinoxalinyl, Chinazolinyl, Cinnolinyl.

Die Erfindung betrifft weiterhin ein Verfahren zur Herstellung der Verbindung I, dadurch gekennzeichnet, daß man eine Verbindung der Formel II 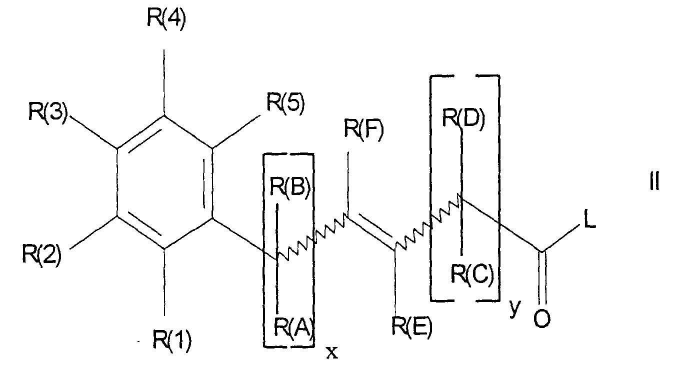 mit Guanidin umsetzt, worin R(1), R(2), R(3), R(4), R(5), R(A), R(B), R(C), R(D), R(E), R(F), x und y die angegebene Bedeutung besitzen und L für eine leicht nucleophil substituierbare Abgangsgruppe steht.

Die aktivierten Säurederivate der Formel II, worin L eine Alkoxy-, vorzugsweise eine Methoxygruppe, eine Phenoxygruppe, Phenylthio-, Methylthio-, 2-Pyridylthiogruppe, einen Stickstoffheterocyclus, vorzugsweise 1-Imidazolyl, bedeutet, erhält man vorteilhaft in an sich bekannter Weise aus den zugrundeliegenden Carbonsäurechloriden (Formel II, L = Cl), die man ihrerseits wiederum in an sich bekannter Weise aus den zugrundeliegenden Carbonsäuren (Formel II, L = OH) beispielsweise mit Thionylchlorid herstellen kann.

Neben den Carbonsäurechloriden der Formel II (L = Cl) lassen sich auch weitere aktivierte Säurederivate der Formel II in an sich bekannter Weise direkt aus den zugrundeliegenden Alkenylsäure-Derivaten (Formel II, L = OH) herstellen, wie beispielsweise die Methylester der Formel II mit L = OCH₃ durch Behandeln mit gasförmigem HCI in Methanol, die Imidazolide der Formel II durch Behandeln mit Carbonyldiimidazol [L = 1-Imidazolyl, Staab, Angew. Chem. Int. Ed. Engl. 1, 351 -367 (1962)], die gemischten Anhydride II mit Cl-COOC₂H₅ oder Tosylchlorid in Gegenwart von Triethylamin in einem inerten Lösungsmittel; möglich sind auch Aktivierungen von Alkenylsäuren mit Dicyclohexylcarbodiimid (DCC) oder mit O-[(Cyano(ethoxycarbonyl)methylen) amino]-1,1,3,3-tetramethyluronium-tetrafluorborat ("TOTU") [Proceedings of the 21. European Peptide Symposium, Peptides 1990, Editors E. Giralt and D. Andreu, Escom, Leiden, 1991]. Eine Reihe geeigneter Methoden zur Herstellung von aktivierten Carbonsäurederivaten der Formel II sind unter Angabe von Quellenliteratur in J. March, Advanced Organic Chemistry, Third Edition (John Wiley & Sons, 1985), S. 350 angegeben.

Die Umsetzung eines aktivierten Carbonsäurederivates der Formel II mit Guanidin erfolgt in an sich bekannter Weise in einem protischen oder aprotischen polaren aber inerten organischen Lösungsmittel. Dabei haben sich bei der Umsetzung der Alkenylsäuremethylester (II, L = OMe) mit Guanidin Methanol, Isopropanol oder THF von 20°C bis zur Siedetemperatur dieser Lösungsmittel bewährt. Bei den meisten Umsetzungen von Verbindungen II mit salzfreiem Guanidin wurde vorteilhaft in aprotischen inerten Lösungsmitteln wie THF, Dimethoxyethan, Dioxan gearbeitet. Aber auch Wasser kann unter Gebrauch einer Base wie beispielsweise NaOH als Lösungsmittel bei der Umsetzung von II mit Guanidin verwendet werden.

Wenn L = Cl bedeutet, arbeitet man vorteilhaft unter Zusatz eines Säurefängers, z. B. in Form von überschüssigen Guanidin zur Abbindung der Halogenwasserstoffsäure.

Ein Teil der zugrundeliegenden Alkenylsäurederivate der Formel II ist bekannt und in der Literatur beschrieben. Die unbekannten Verbindungen der Formel II können nach literaturbekannten Methoden hergestellt werden. Die erhaltenen Alkenylsäuren werden nach einer der oben beschriebenen Verfahrensvarianten zu erfindungsgemäßen Verbindungen I umgesetzt.

Die Einführung einiger Substituenten gelingt durch literaturbekannte Methoden des Palladium-vermittelten cross-couplings von Arylhalogeniden bzw. Aryltriflaten mit beispielsweise Organostannanen, Organoboronsäuren oder Organoboranen oder Organokupfer- bzw. -zink-verbindungen.

Alkenylguanidine I sind im allgemeinen schwache Basen und können Säure unter Bildung von Salzen binden. Als Säureadditionssalze kommen Salze aller pharmakologisch verträglichen Säuren in frage, beispielsweise Halogenide, insbesondere Hydrochloride, Lactate, Sulfate, Citrate, Tartrate, Acetate, Phosphate, Methylsulfonate, p-Toluolsulfonate.

Die Verbindungen der Formel I sind substituierte Acylguanidine. Prominentester Vertreter der Acylguanidine sind Pyrazincarbonsäure- und Benzoesäureguanidide der allgemeinen Formeln V und VI. 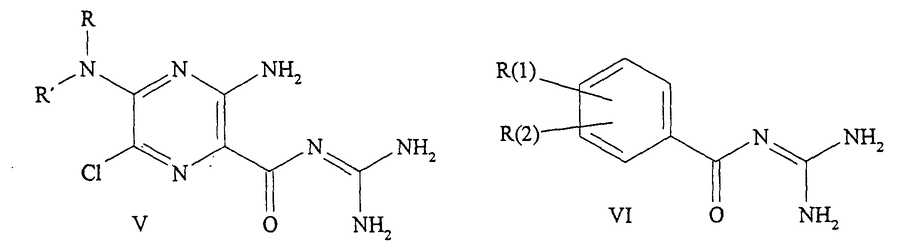
Amilorid: R, R'= H
Dimethylamilorid: R, R'= CH₃
Ethylisopropylamilorid:R =CH(CH₃)₂, R'= C₂H₅

Verbindungen der Formel V werden in der Literatur allgemein als Amiloride bezeichnet. Amilorid=selbst (R, R' = H) findet als kaliumsparendes Diuretikum in der Therapie Verwendung. Zahlreiche weitere Verbindungen vom Amilorid-Typ werden in der Literatur beschrieben, wie beispielsweise Dimethylamilorid oder Ethylisopropylamilorid.

Es existieren Untersuchungen, die auf antiarrhythmische Eigenschaften von Amilorid hinweisen [Circulation 79, 1257 -63 (1989)]. Einer breiten Anwendung als Antiarrhythmikum steht jedoch entgegen, daß dieser Effekt nur schwach ausgeprägt ist und von einer blutdrucksenkenden und saluretischen Wirkung begleitet auftritt und diese Nebenwirkungen bei der Behandlung von Herz-Rhythmusstörungen unerwünscht sind.

Hinweise auf antiarrhythmische Eigenschaften des Amilorids wurden auch bei Experimenten an isolierten Tierherzen erhalten [Eur. Heart J. 9 (suppl.1): 167 (1988) (book of abstracts)]. So wurde beispielsweise an Rattenherzen gefunden, daß ein künstlich ausgelöstes Kammerflimmern durch Amilorid völlig unterdrückt werden konnte. Noch potenter als Amilorid war in diesem Modell das oben erwähnte Amiloridderivat Ethylisopropylamilorid.

Die Verbindungen der Formel VI sind selektive Inhibitoren des ubiquitären Natrium/Protonen-Austauschers (Subtyp 1, NHE-1). Literaturbekannte Vertreter sind das HOE 694 und das HOE 642, die als antiarrhythmisch und unter ischämischen Bedingungen als cardioprotektiv beschrieben werden [ a) Scholz W, Albus U, Linz W, Martorana P, Lang HJ, Schölkens BA. Effects of Na⁺/H⁺ exchange inhibitors in cardiac ischemia. J Mol Cell Cardiol 1992;24:731-739; b) Scholz W, Albus U. Na⁺/H⁺ exchange and its inhibition in cardiac ischemia and reperfusion. Basic Res Cardiol 1993;88:443-455; c) Scholz W, Albus U, Counillon L, Gögelein H, Lang HJ, Linz W, Weichert A, Schölkens BA. Protective effects of HOE 642, a selective sodium-hydrogen exchange subtype 1 inhibitor, on cardiac ischaemia and reperfusion. Cardiovasc Res 1995;29:260 - 268; d) Bugge, E. and Ytrehus, K. Inhibition of sodium-hydrogen exchange reduces infarct size in the isolated rat heart -- A protective additive to ischaemic preconditioning. Cardiovasc. Res. 29:269-274, 1995. ].

Weiterhin sind 3-Phenyl- und 3-Thiophenyl-propensäureguanidide VII als NHE-lnhibitoren literaturbekannt [ US 2 734 904, WO 84/00875, DE-OS 44 21 536.3 (HOE 94/F 168)] jedoch sind in diesen Veröffentlichungen keine Bisguanidinverbindungen der Formel I beschrieben oder nahegelegt.

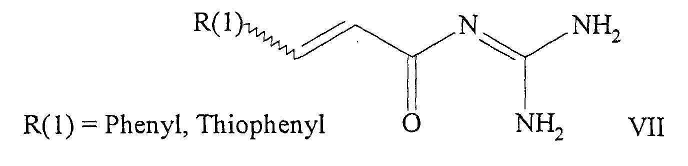

Überraschenderweise inhibieren die erfindungsgemäßen Verbindungen der Formel I den Natrium/Protonen-Austauscher Subtyp 3. Die bisher bekannten NHE-Inhibitoren sind an diesem Subtyp kaum aktiv.

Die beanspruchten Verbindungen der Formel I wirken blutdrucksenkend und eignen sich somit als Arzneimittel zur Behandlung der primären und sekundären Hypertonie. Sie eignen sich aufgrund ihrer salidiuretischen Wirkung als Diuretika.

Außerdem wirken die Verbindungen alleine oder in Verbindung mit NHE-lnhibitoren anderer Subtypspezifität antiischämisch. Sie schützen akut oder chronisch sauerstoffmangelversorgte Organe durch Verringerung oder Verhinderung ischämisch induzierter Schäden und eignen sich somit als Arzneimittel beispielsweise bei Thrombosen, Gefäßspasmen, Atherosklerose oder bei operativen Eingriffen (z.B. bei Organ-Transplantationen von Niere und Leber, wobei die Verbindungen sowohl für den Schutz der Organe im Spender vor und während der Entnahme, zum Schutz entnommener Organe beispielsweise bei Behandlung mit oder deren Lagerung in physiologischen Badflüssigkeiten, wie auch bei der Überführung in den Empfängerorganismus verwendet werden können) oder chronischem oder akutem Nierenversagen.

Entsprechend ihrer protektiven Wirkung gegen ischämisch induzierte Schäden sind die Verbindungen auch als Arzneimittel zur Behandlung von Ischämien des Nervensystems, insbesondere des ZNS, geeignet, wobei sie z.B. zur Behandlung des Schlaganfalls oder des Hirnödems geeignet sind. Darüber hinaus eignen sich die erfindungsgemäßen Verbindungen der Formel I ebenfalls zur Behandlung von Formen des Schocks, wie beispielweise des allergischen, cardiogenen, hypovolämischen und des bakteriellen Schocks.

Weiterhin induzieren die Verbindungen eine Verbesserung des Atemantriebes und werden deshalb zur Behandlung von Atmungszuständen bei folgenden klinischen Zuständen und Krankheiten herangezogen: Gestörter zentraler Atemantrieb (z.B. zentrale Schlafapnoen, plötzlicher Kindstod, postoperative Hypoxie), muskulär-bedingte Atemstörungen, Atemstörungen nach Langzeitbeatmung, Atemstörungen bei Adaptation im Hochgebirge, obstruktive und gemischte Form der Schlafapnoen, akute und chronische Lungenkrankheiten mit Hypoxie und Hyperkapnie.

Eine Kombination eines NHE-Inhibitors mit einem Carboanhydrase-Hemmer (z.B. Acetazolamid), wobei letzterer eine metabolische Azidose herbeiführt und dadurch bereits die Atmungstätigkeit steigert, erweist sich als günstige Kombination mit verstärkter Wirkung und vermindertem Wirkstoffeinsatz.

Darüber hinaus zeichnen sich die erfindungsgemäßen Verbindungen der Formel I durch starke inhibierende Wirkung auf die Proliferationen von Zellen, beispielsweise der Fibroblasten- Zellproliferation und der Proliferation der glatten Gefäßmuskelzellen, aus. Deshalb kommen die Verbindungen der Formel I als wertvolle Therapeutika für Krankheiten in Frage, bei denen die Zellproliferation eine primäre oder sekundäre Ursache darstellt, und können deshalb als Antiatherosklerotika, Mittel gegen diabetische Spätkomplikationen, Krebserkrankungen, fibrotische Erkrankungen wie Lungenfibrose, Leberfibrose oder Nierenfibrose, endotheliale Disfunktion, Organhypertrophien und -hyperplasien, insbesondere bei Prostatahyperplasie bzw. Prostatahypertrophie verwendet werden. Außerdem bewirken die Verbindungen eine Senkung des Cholesterinspiegels und sind dadurch als Arzneimittel zur Prävention und Behandlung der Atherosklerose geeignet.

Die erfindungsgemäßen Verbindungen sind wirkungsvolle Inhibitoren des zellulären Natrium-Protonen-Antiporters (Na⁺/H⁺-Exchanger), Subtyp 1 und 3, der bei zahlreichen Erkrankungen (Essentielle Hypertonie, Atherosklerose, Diabetes usw.) auch in solchen Zellen erhöht ist, die Messungen leicht zugänglich sind, wie beispielsweise in Erythrocyten, Thrombocyten oder Leukozyten. Die erfindungsgemäßen Verbindungen eignen sich deshalb als hervorragende und einfache wissenschaftliche Werkzeuge, beispielsweise in ihrer Verwendung als Diagnostika zur Bestimmung und Unterscheidung bestimmter Formen der Hypertonie, aber auch der Atherosklerose, des Diabetes, proliferativer Erkrankungen usw.. Darüber hinaus sind die Verbindungen der Formel I für die präventive Therapie zur Verhinderung der Genese des Bluthochdrucks, beispielweise der essentiellen Hypertonie, geeignet.

Es wurde außerdem gefunden, daß Verbindungen der Formel I eine günstige Beeinflussung der Serumlipoproteine zeigen. Es ist allgemein anerkannt, daß für die Entstehung arteriosklerotischer Gefäßveränderungen, insbesondere der koronaren Herzkrankheit, zu hohe Blutfettwerte, sogenannte Hyperlipoproteinämien, einen wesentlichen Risikofaktor darstellen. Für die Prophylaxe und die Regression von atherosklerotischen Veränderungen kommt daher der Senkung erhöhter Serum-Lipoproteine eine außerordentliche Bedeutung zu. Neben der Reduktion des Serum-Gesamtcholesterins kommt der Senkung des Anteils spezifischer atherogener Lipidfraktionen dieses Gesamtcholesterins, insbesondere der low density Lipoproteine (LDL) und der very low density Lipoproteine (VLDL) eine besondere Bedeutung zu, da diese Lipidfraktionen einen atherogenen Risikofaktor darstellen. Dagegen wird den high density Lipoproteinen eine Schutzfunktion gegen die koronare Herzkrankheit zugeschrieben. Dementsprechend sollen Hypolipidämika in der Lage sein, nicht nur das Gesamtcholesterin, sondern insbesondere die VLDL und LDL-Serumcholesterinfraktionen zu senken. Es wurde nun gefunden, daß Verbindungen der Formel I bezüglich der Beeinflussung der Serumlipidspiegel wertvolle therapeutisch verwertbare Eigenschaften zeigen. So erniedrigen sie signifikant die erhöhten Serum Konzentration von LDL und VLDL, wie sie beispielweise durch erhöhte dietätische Einnahme einer cholesterin- und lipidreichen Kost oder bei pathologischen Stoffwechselveränderungen, beispielsweise genetisch bedingten Hyperlipidämien zu beobachten sind. Sie können daher zur Prophylaxe und zur Regression von atherosklerotischen Veränderungen herangezogen werden, indem sie einen kausalen Risikofaktor ausschalten. Hierzu zählen nicht nur die primären Hyperlipidämien, sondern auch gewisse sekundäre Hyperlipidämien, wie sie z.B. beim Diabetes vorkommen. Darüber hinaus führen die Verbindungen der Formel I zu einer deutlichen Reduktion der durch Stoffwechselanomalien induzierten Infarkte und insbesondere zu einer signifikanten Verminderung der induzierten Infarktgröße und dessen Schweregrades. Weiterhin führen Verbindungen der Formel I zu einen wirksamen Schutz gegen durch Stoffwechselanomalien induzierte Endothelschädigungen. Mit diesem Schutz der Gefäße gegen das Syndrom der endothelialen Dysfunktion sind Verbindungen der Formel I wertvolle Arzneimittel zur Prävention und zur Behandlung koronarer Gefäßspasmen, der Atherogenese und der Atherosklerose, der linksventrikulären Hypertrophie und der dilatierten Kardiomyopathie, und thrombotischer Erkrankungen.

Die genannten Verbindungen finden deshalb vorteilhaft Verwendung zur Herstellung eines Medikaments zur Behandlung von Hypercholesterinämie; zur Herstellung eines Medikaments zur Prävention der Atherogenese; zur Herstellung eines Medikaments zur Prävention und Behandlung der Atherosklerose, zur Herstellung eines Medikaments zur Prävention und Behandlung von Krankheiten, die durch erhöhte Cholesterinspiegel ausgelöst werden, zur Herstellung eines Medikaments zur Prävention und Behandlung von Krankheiten; die durch endotheliale Dysfunktion ausgelöst werden, zur Herstellung eines Medikaments zur Prävention und Behandlung von atherosklerose-induzierter Hypertonie, zur Herstellung eines Medikaments zur Prävention und Behandlung von atherosklerose-induzierter Thrombosen, zur Herstellung eines Medikaments zur Prävention und Behandlung von Hypercholesterinämie- und endothelialer Dysfunktion induzierter ischämischer Schäden und postischämischer Reperfusionsschäden, zur Herstellung eines Medikaments zur Prävention und Behandlung von Hypercholesterinämie- und endothelialer Dysfunktion induzierter cardialer Hypertrophien und Cardiomyopathien, zur Herstellung eines Medikaments zur Prävention und Behandlung von Hypercholesterinämie- und endothelialer Dysfunktion induzierter koronarer Gefäßspasmen und myocardialer Infarkte, zur Herstellung eines Medikaments zur Behandlung der genannten Leiden in Kombinationen mit blutdrucksenkenden Stoffen, bevorzugt mit Angiotensin Converting Enzyme (ACE)-Hemmern und Angiotensin-Rezeptorantagonisten. Eine Kombination eines NHE-Inhibitors der Formel I mit einem blutfettspiegelsenkenden Wirkstoff, bevorzugt mit einem HMG-CoA-Reduktaseinhibitor (z.B. Lovastatin oder Pravastatin), wobei letzterer eine hypolipidämische Wirkung herbeiführt und dadurch die hypolipidämischen Eigenschaften des NHE-Inhibitors der Formel I steigert, erweist sich als günstige Kombination mit verstärkter Wirkung und vermindertem Wirkstoffeinsatz.

Beansprucht wird die Gabe von Natrium-Protonen-Austausch-Hemmern der Formel I als neuartige Arzneimittel zur Senkung erhöhter Blutfettspiegel, sowie die Kombination von Natrium-Protonen-Austausch-Hemmern mit blutdrucksenkenden und/oder hypolipidämisch wirkenden Arzneimitteln.

Arzneimittel, die eine Verbindung I enthalten, können dabei oral, parenteral, intravenös, rektal oder durch Inhalation appliziert werden, wobei die bevorzugte Applikation von dem jeweiligen Erscheinungsbild der Erkrankung abhängig ist. Die Verbindungen I können dabei allein oder zusammen mit galenischen Hilfsstoffen zur Anwendung kommen, und zwar sowohl in der Veterinär-als auch in der Humanmedizin.

Welche Hilfsstoffe für die gewünschte Arzneimittelformulierung geeignet sind, ist dem Fachmann auf Grund seines Fachwissens geläufig. Neben Lösemitteln, Gelbildnern, Suppositorien-Grundlagen, Tablettenhilfsstoffen und anderen Wirkstoffträgern können beispielsweise Antioxidantien, Dispergiermittel, Emulgatoren, Entschäumer, Geschmackskorrigentien, Konservierungsmittel, Lösungsvermittler oder Farbstoffe verwendet werden.

Für eine orale Anwendungsform werden die aktiven Verbindungen mit den dafür geeigneten Zusatzstoffen, wie Trägerstoffen, Stabilisatoren oder inerten Verdünnungsmittel vermischt und durch die üblichen Methoden in die geeigneten Darreichungsformen gebracht, wie Tabletten, Dragees, Steckkapseln, wäßrige, alkoholische oder ölige Lösungen. Als inerte Träger können z. B. Gummi arabicum, Magnesia, Magnesiumcarbonat, Kaliumphosphat, Milchzucker, Glucose oder Stärke, insbesondere Maisstärke, verwendet werden. Dabei kann die Zubereitung sowohl als Trocken- als auch als Feuchtgranulat erfolgen. Als ölige Trägerstoffe oder als Lösemittel kommen beispielsweise pflanzliche oder tierische Öle in Betracht, wie Sonnenblumenöl oder Lebertran.

Zur subkutanen oder intravenösen Applikation werden die aktiven Verbindungen, gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel kommen z. B. in Frage: Wasser, physiologische Kochsalzlösung oder Alkohole, z. B. Ethanol, Propanol, Glycerin, daneben auch Zuckerlösungen wie Glucose-oder Mannitlösungen, oder auch eine Mischung aus den verschiedenen genannten Lösungsmitteln.

Als pharmazeutische Formulierung für die Verabreichung in Form von Aerosolen oder Sprays sind geeignet z. B. Lösungen, Suspensionen oder Emulsionen des Wirkstoffes der Formel I in einem pharmazeutisch unbedenklichen Lösungsmittels, wie insbesondere Ethanol oder Wasser, oder einem Gemisch solcher Lösungsmittel.

Die Formulierung kann nach Bedarf auch noch andere pharmazeutische Hilfsstoffe wie Tenside, Emulgatoren und Stabilisatoren sowie ein Treibgas enthalten. Eine solche Zubereitung enthält den Wirkstoff üblicherweise in einer Konzentration von etwa 0,1 bis 10, insbesondere von etwa 0,3 bis 3 Gew.-%.

Die Dosierung des zu verabreichenden Wirkstoffs der Formel I und die Häufigkeit der Verabreichung hängen von der Wirkstärke und Wirkdauer der verwendeten Verbindungen ab; außerdem auch von Art und Stärke der zu behandelnden Krankheit sowie von Geschlecht, Alter, Gewicht und individueller Ansprechbarkeit des zu behandelnden Säugers. Im Durchschnitt beträgt die tägliche Dosis einer Verbindung der Formel I bei einem etwa 75 kg schweren Patienten mindestens 0,001 mg/kg, vorzugsweise 0,01 mg/kg, bis höchstens 10 mg/kg, vorzugsweise 1 mg/kg Körpergewicht. Bei akuten Ausbrüchen der Krankheiten können auch noch höhere und vor allem häufigere Dosierungen notwendig sein, z. B. bis zu 4 Einzeldosen pro Tag. Insbesondere bei i.v. Anwendung, etwa bei einem Infarktpatienten auf der Intensivstation können bis zu 200 mg pro Tag notwendig werden.

### Liste der Abkürzungen:

- MeOH: Methanol
- DMF: N,N-Dimethylformamid
- El: electron impact
- DCI: Desorption-Chemical Ionisation
- RT: Raumtemperatur
- EE: Ethylacetat (EtOAc)
- mp: Schmelzpunkt
- HEP: n-Heptan
- DME: Dimethoxyethan
- ES: Elektronenspray
- FAB: Fast Atom Bombardment
- CH₂Cl₂: Dichlormethan
- THF: Tetrahydrofuran
- eq.: Äquivalent

### Experimenteller Teil

Allgemeine Vorschriften zur Herstellung von Alkenylcarbonsäure-guanidinen (I) Variante 1 A: aus Alkenylcarbonsäuren (II, L=OH)
1.0 eq. des Carbonsäurederivates der Formel II löst bzw. suspendiert man in wasserfreiem THF ( 5 ml/mmol) und versetzt sodann mit 1.1 eq. Carbonyldiimidazol. Nach dem Rühren über 2 Stunden bei RT werden 5.0 eq. Guanidin in die Reaktionslösung eingetragen. Nach dem Rühren über Nacht destilliert man das THF unter vermindertem Druck (Rotationsverdampfer) ab, versetzt mit Wasser, stellt mit 2 N HCl auf pH 6 bis 7 und filtriert das entsprechende Guanidid (Formel I) ab. Die so erhaltenen Carbonsäureguanidine können durch Behandeln mit wäßriger, methanolischer oder etherischer Salzsäure oder anderen pharmakologisch verträglichen Säuren in die entsprechenden Salze übergeführt werden.

Variante 1 B: aus Alkenylcarbonsäure-alkylestern (II, L=O-Alkyl 1.0 eq. des Carbonsäure-alkylesters der Formel II sowie 5.0 eq. Guanidin (freie Base) werden in Isopropanol gelöst oder in THF suspendiert und bis zum vollständigen Umsatz (Dünnschichtkontrolle) unter Rückfluß gekocht (typische Reaktionszeit 2 bis 5 h). Das Lösungsmittel wird unter vermindertem Druck (Rotationsverdampfer) abdestilliert, in EE aufgenommen und 3 x mit NaHCO₃-Lösung gewaschen. Es wird über Na₂SO₄ getrocknet, das Lösungsmittel im Vakuum abdestilliert und an Kieselgel mit einem geeigneten Laufmittel, z. B. EE/MeOH 5 : 1 chromatographiert. (Salzbildung vergleiche Variante A)

### Beispiel 1: 1,2-Bis-[3-(E-2-methyl-propensäureguanidid)]benzol-dihydrochlorid

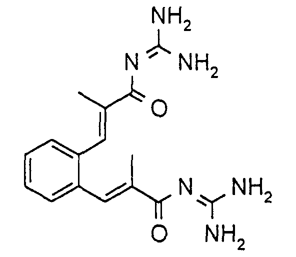

1 a) 1 eq. 2-Phosphonopropionsäuretriethylester wurde bei 0°C mit 1 eq. n-Butyllithium in Hexan deprotoniert und anschließend bei RT mit 0.5 eq. 1,2-Phthaldialdehyd versetzt. Nach vollständiger Abreaktion des Dialdehyds wurde mit Wasser aufgearbeitet und dreimal mit Toluol ausgeschüttelt. Nach Trocknen der vereinigten organischen Phasen über Magnesiumsulfat wurde das Lösungsmittel im Vakuum entfernt und das verbleibende Rohprodukt chromatographisch an Kieselgel mit EE/HEP-Gemischen als Eluens getrennt. Man isolierte: 1,2-Di-[3-(E-2-methyl-propensäureethylester)]-benzol.
   Farbloses Öl; MS MS (DCI):303 (M+1)⁺
1 b) Der Ester aus 1 a) wurde gemäß einer Standardmethode (Natrium-hydroxid in Methanol) verseift. Man isolierte : 1,2-Di-[3-(E-2-methyl-propensäure)]-benzol. farbloser Feststoff;
   mp: >187°C MS (DCI):246 (M⁺)
1 c) Die Dicarbonsäure aus 1 b) wurde gemäß Variante 1 A ins Diguanidid-dihydrochlorid überführt.
   farbloser Feststoff;
   mp: >200°C; MS (DCI):329 (M+1)⁺

### Beispiel 2: 1,3-Bis-[3-(E-2-methyl-propensäureguanidid)]benzol-dihydrochlorid

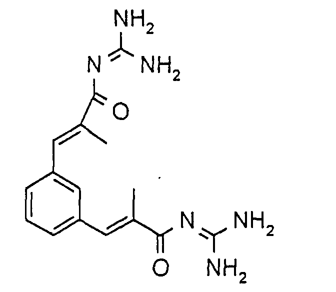

2 a) 1 eq. 2-Phosphonopropionsäuretriethylester wurde bei 0°C mit 1 eq. n-Butyllithium in Hexan deprotoniert und anschließend bei RT mit 0.5 eq. 1,3-Isophthaldialdehyd versetzt. Nach vollständiger Abreaktion des Dialdehyds wurde mit Wasser aufgearbeitet und dreimal mit Toluol ausgeschüttelt. Nach Trocknen der vereinigten organischen Phasen über Magnesiumsulfat wurde das Lösungsmittel im Vakuum entfernt und das verbleibende Rohprodukt chromatographisch an Kieselgel mit EE/HEP-Gemischen als Eluens getrennt. Man isolierte: 1,3-Di-[3-(E-2-methyl-propensäureethylester)]-benzol.
   farbloses Öl; MS MS (DCI):303 (M+1⁺)
2 b) Der Ester aus 2 a) wurde gemäß einer Standardmethode (Natrium-hydroxid in Methanol) verseift. Man isolierte: 1,3-Di-[3-(E-2-methyl-propensäure)]-benzol. farbloser Feststoff;
   mp >190°C; MS (DCI): 247 (M+1⁺)
2 c) Die Dicarbonsäure aus 2 b) wurde gemäß Variante 1 A ins Diguanidid-dihydrochlorid überführt.
   farbloser Feststoff;
   mp: 175°C; MS (DCI):329 (M+1)⁺

### Beispiel 3: 1,4-Bis-[3-(E-2-methyl-propensäureguanidid)]benzol-dihydrochlorid

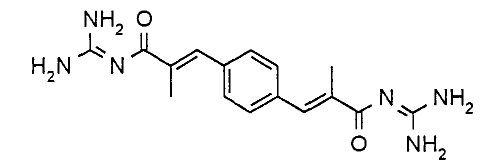

3 a) 1 eq. 2-Phosphonopropionsäuretriethylester wurde bei 0°C mit 1 eq. n-Butyllithium in Hexan deprotoniert und anschließend bei RT mit 0.5 eq. 1,4-Terephthaldialdehyd versetzt. Nach vollständiger Abreaktion des Dialdehyds wurde mit Wasser aufgearbeitet und dreimal mit Toluol ausgeschüttelt. Nach Trocknen der vereinigten organischen Phasen über Magnesiumsulfat wurde das Lösungsmittel im Vakuum entfernt und das verbleibende Rohprodukt chromatographisch an Kieselgel mit EE/HEP-Gemischen als Eluens getrennt. Man isolierte: 1,4-Di-[3-(E-2-methyl-propensäureethylester)]-benzol.
   farbloser Feststoff;
   mp:41°C; MS (DCI):303 (M+1⁺)
3 b) Der Ester aus 3 a) wurde gemäß einer Standardmethode (Natrium-hydroxid in Methanol) verseift. Man isolierte: 1,4-Di-[3-(E-2-methyl-propensäure)]-benzol. farbloser Feststoff;
   mpi>190°C; MS (DCI): 247 (M+1⁺)
3 c) Die Dicarbonsäure aus 3 b) wurde gemäß Variante 1 A ins Diguanidid-dihydrochlorid überführt.
   gelber Feststoff;
   mp: 255°C; MS (DCI):329 (M+1⁺)

### Beispiel 4: 2,3-Bis-[3-(E-2-methyl-propensäureguanidid)]naphthalin-dihydrochlorid

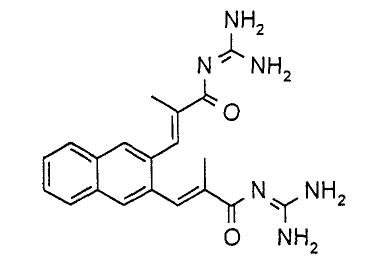

4 a) 1 eq. 2-Phosphonopropionsäuretriethylester wurde bei 0°C mit 1 eq. n-Butyllithium in Hexan deprotoniert und anschließend bei RT mit 0.5 eq. 2,3-Naphthalindialdehyd versetzt. Nach vollständiger Abreaktion des Dialdehyds wurde mit Wasser aufgearbeitet und dreimal mit Toluol ausgeschüttelt. Nach Trocknen der vereinigten organischen Phasen über Magnesiumsulfat wurde das Lösungsmittel im Vakuum entfernt und das verbleibende Rohprodukt chromatographisch an Kieselgel mit EE/HEP-Gemischen als Eluens getrennt. Man isolierte: 2,3-Di-[3-(E-2-methyl-propensäureethylester)]-naphthalin.
   farbloses Öl; MS (DCI):353 (M+1⁺)
4 b) Der Ester aus 4 a) wurde gemäß einer Standardmethode (Natrium-hydroxid in Methanol) verseift. Man isolierte: 2,3-Di-[3-(E-2-methyl-propensäure)]-naphthalin.
   farbloser Feststoff;
   mp: >210°C; MS (DCI): 295 (M-H)⁻
4 c) Die Dicarbonsäure aus 4 b) wurde gemäß Variante 1 A ins Diguanidid-dihydrochlorid überführt.
   farbloser Feststoff;
   mp: >200°C; MS (DCI):379 (M+1)⁺

### Beispiel 5: 1,2-Bis-[3-(Z-2-fluor-propensäureguanidid)]benzol-dihydrochlorid

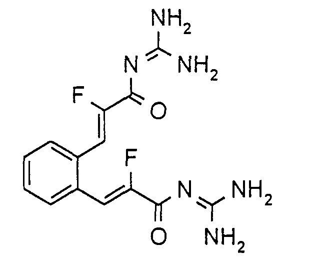

5 a) In Anlehnung an ein literaturbekanntes Verfahren (Cousseau et al., Tetrahedron Letters 34, 1993, 6903) wurde ausgehend vom 1,2-Phthaldialdehyd das 1,2-Di-[3-(Z-2-fluor-propensäureethylester)]benzol dargestellt und an Kieselgel mit EE/HEP-Gemischen als Eluens gereinigt und isoliert.
   farbloser Feststoff
   mp: amorph; MS (DCI):311 (M+1)⁺
5 b) Der Diester aus 5 a) wurde gemäß Variante 1 B zum Diguanidid umgesetzt und ins Dihydrochlorid überführt.
   mp.: >235°C; MS (DCI):337 (M+1)⁺

### Beispiel 6: 1-[3-(Z-2-fluor-propensäureguanidid)]-2-[3-(E-2-methyl-propensäureguanidid)]benzol-dihydrochlorid

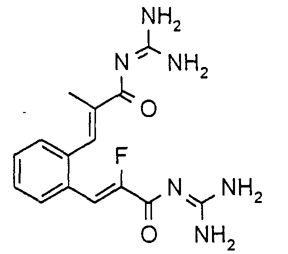

6 a) Der bei der Darstellung von 7 a) ebenfalls isolierte Monoaldehyd-monoester 6 a) wurde in Anlehnung an 4 a) in den Diester 6 b) überführt.

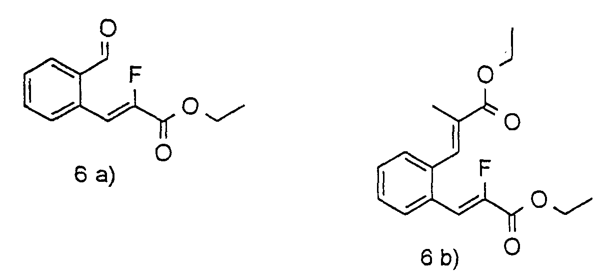

| | |
|---|---|
| gelbliches Öl | gelbliches Öl |
| MS (CI): 223 (M+1)⁺ | MS (Cl): 307 (M+1)⁺ |

6 c) Der Diester aus 6 b) wurde gemäß Variante 1 B zum Diguanidid umgesetzt und ins Dihydrochlorid überführt.
Schmp.: >200°C; MS (ES):333 (M+1)⁺

### Beispiel 7: 1,3-Bis-[3-(Z-2-fluor-propensäureguanidid)]benzol-dihydrochlorid

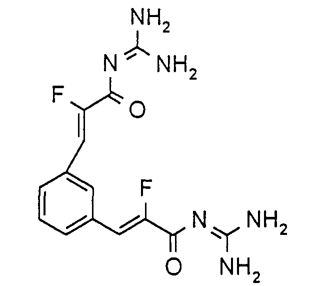

7 a) In Anlehnung an ein literaturbekanntes Verfahren (Cousseau et al., Tetrahedron Letters 34, 1993, 6903) wurde ausgehend vom 1,3-Isophthaldialdehyd das 1,3-Di-[3-(Z-2-fluor-propensäureethylester)]benzol dargestellt und an Kieselgel mit EE/HEP-Gemischen als Eluens gereinigt und isoliert.
   farbloser Feststoff
   mp: amorph; MS (CI):311 (M+1)⁺
7 b) Der Diester aus 7 a) wurde gemäß Variante 1 B zum Diguanidid umgesetzt und ins Dihydrochlorid überführt.
   orange/gelbfarbener Feststoff;
   Schmp.:>180°C; MS (ES):337 (M+1)⁺

### Beispiel 8: 3-(4-Chlor-3-guanidinocarbonyl-5-phenyl)phenyl-2-methyl-propensäureguanidid

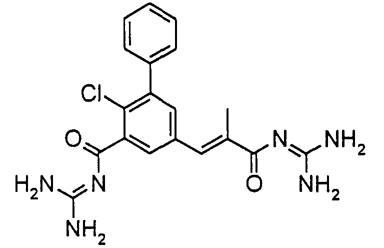

8 a) 3-Brom-2-chlor-5-methyl-benzoesäure
   25 g 2-Amino-3-brom-5-methyl-benzoesäure wurden in 500 ml 6 N wäßriger HCI-Lösung gelöst, bei 0°C mit 8.25 g NaNO₂ versetzt und 30 Minuten bei dieser Temperatur diazotiert. Diese Diazoniumsalzlösung wurde anschließend auf eine 40°C warme Lösung von 22 g CuCl in 200 ml einer gesättigten wäßrigen HCI-Lösung getropft und 20 Minuten bei dieser Temperatur nachgerührt. Das Produkt wurde abgesaugt, mit 500 ml Wasser gewaschen und bei 40°C im Feinvakuum getrocknet. Man erhielt 23.3 g weißer Kristalle,
   mp 170 - 172°C; R_{f} (EE/MeOH 5:1) = 0.51; MS (DCI) 249 (M+H)⁺
8 b) 3-Brom-2-chlor-5-dibrommethyl-benzoesäure
   10g 3-Brom-2-chlor-5-methyl-benzoesäure wurden in 150 ml Chlorbenzol gelöst und zum Rückfluß erhitzt. Bei dieser Temperatur wurde zunächst 7.2 g N-Bromsuccinimid und 0.5 g Benzoylperoxid zugegeben und unter Rückfluß 30 Minuten gekocht. Anschließend wurde ein zweites Mal 7.2 g N-Bromsuccinimid und 0.5 g Benzoylperoxid zugegeben und weitere 3 Stunden unter Rückfluß gekocht. Nach dem Abkühlen wurden 200 ml EE zugegeben, mit 50 ml gesättigter wäßriger Na₂SO₃-Lösung/300 ml gesättigter wäßriger KH₂PO₄-Lösung gewaschen und die wäßrige Phase 2 mal mit je 200 ml EE extrahiert. Über Na₂SO₄ wurde getrocknet und das Solvens im Vakuum entfernt. Man erhielt 14.1 g eines gelben Öls.
   R_{f} (DIP/2% HOAc) = 0.32
8 c) 3-Brom-2-chlor-5-formyl-benzoesäure
   11.7 g AgNO₃ wurden in 150 ml Wasser und 150 ml MeOH gelöst und eine Lösung aus 14 g 3-Brom-2-chlor-5-dibrommethyl-benzoesäure in 100 ml MeOH zugetropft. 30 Minuten wurde bei RT nachgerührt, 100 ml einer gesättigten wäßrigen NaCI-Lösung zugegeben, die Silbersalze abgesaugt und die Solventien im Vakuum entfernt. Der Rückstand wurde mit 200 ml einer 5% wäßrigen KHSO₄-Lösung aufgenommen und 3 mal mit je 200 ml EE extrahiert. Über Na₂SO₄ wurde getrocknet und das Solvens im Vakuum entfernt. Chromatographie an Kieselgel mit DIP/2% HOAc lieferte 3.6 g farbloser Kristalle,
   mp: 148°C; R_{f} (DIP/2% HOAc) = 0.12; MS (DCI) 263 (M+H)⁺
8 d) 3-Brom-2-chlor-5-formyl-benzoesäureethylester
   3.6 g 3-Brom-2-chlor-5-formyl-benzoesäure wurden in 100 ml EtOH gelöst, 2.9 ml SOCl₂ zugetropft und 5 Stunden unter Rückfluß gekocht. Anschließend wurden die flüchtigen Bestandteile im Vakuum entfernt und der Rückstand an Kieselgel mit EE/HEP 1:8 chromatographiert. Man erhielt 2.7 g eines farblosen Öls.
   R_{f} (EE/HEP 1:8) = 0.24; MS (DCI) : 291 (M+H)⁺
8 e) 3-(3-Brom-4-chlor-5-ethoxycarbonyl)phenyl-2-methyl-propensäure-ethylester
   2.7 g 3-Brom-2-chlor-5-formyl-benzoesäureethylester wurden analog Beispiel 4 a) einer Wittig-Horner Reaktion unterzogen, und man erhielt 3.4 g eines farblosen Öls.
   R_{f} (EE/HEP 1:8) = 0.27; MS (DCI) : 374 (M+H)⁺
8 f) 3-(4-Chlor-3-ethoxycarbonyl-5-phenyl)phenyl-2-methyl-propensäure-ethylester
   3.3 g 3-(3-Brom-4-chlor-5-ethoxycarbonyl)phenyl-2-methyl-propensäure-ethylester, 1.23 g Phenylboronsäure, 2.14 g Na₂CO₃, 576 mg Triphenylphosphin und 227 mg Pd(OAc)₂ wurden in 150 ml Toluol und 40 ml Wasser gelöst und 7 Stunden unter Rückfluß gekocht. Anschließend wurde auf RT abgekühlt, mit 200 ml EE versetzt und 2 mal mit je 100 ml einer gesättigten wäßrigen Na₂CO₃-Lösung sowie mit 100 ml einer gesättigten wäßrigen NaCI-Lösung gewaschen, über Na₂SO₄ getrocknet und das Solvens im Vakuum entfernt. Chromatographie an Kieselgel mit EE/HEP 1:8 lieferte 800 mg eines farblosen Öls.
   R_{f} (EE/HEP 1:8) = 0.25; MS (DCI): 372 (M+H)⁺
8 g) 3-(4-Chlor-3-guanidinocarbonyl-5-phenyl)phenyl-2-methyl-propensäureguanidid
   1.8 g Kalium-t-butylat wurden in 100 ml DMF gelöst, 1.82 g Guanidin-hydrochlorid hinzugefügt und 1 Stunde bei RT gerührt. Anschließend wurden 700 mg 3-(4-Chlor-3-ethoxycarbonyl-5-phenyl)phenyl-2-methyl-propensäure-ethylester zugegeben und 5 Stunden bei 100°C gerührt. Das Reaktionsgemisch wurde auf 200 ml Wasser gegossen und 3 mal mit je 200 ml EE extrahiert. Über Na₂SO₄ wurde getrocknet und das Solvens im Vakuum entfernt. Chromatographie an Kieselgel mit Aceton/Wasser 10: 1 lieferte 170 mg eines amorphen Schaums.
   R_{f} (Aceton/Wasser 10:1) = 0.23; MS (FAB) : 399 (M+H)⁺

### Beispiel 9: 1,3-Bis-[3-(E-2-methyl-propensäureguanidid)]-2-methoxy-5-methyl-benzol-hydrochlorid

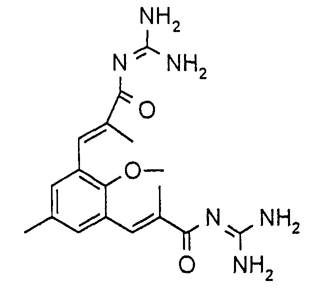

9 a) 1 eq. 2-Phosphonopropionsäuretriethylester wurde bei 0°C mit 1 eq. n-Butyllithium in Hexan deprotoniert und anschließend bei RT mit 0.5 eq. 2-Methoxy-5-methyl-1,3-isophthaldialdehyd versetzt. Nach vollständiger Abreaktion des Dialdehyds wurde mit Wasser aufgearbeitet und dreimal mit Toluol ausgeschüttelt. Nach Trocknen der vereinigten organischen Phasen über Magnesiumsulfat wurde das Lösungsmittel im Vakuum entfernt und das verbleibende Rohprodukt chromatographisch an Kieselgel mit EE/HEP-Gemischen als Eluens getrennt. Man isolierte: 1,3-Di-[3-(E-2-methyl-propensäureethylester)]-2-methoxy-5-methyl -benzol.
   farbloses Öl; MS (CI):347 (M+1⁺)
9 b) Der Diester aus 9 a) wurde gemäß einer Standardmethode (Natrium-hydroxid in Methanol) verseift. Man isolierte: 1,3-Di-[3-(E-2-methyl-propensäure)]-2-methoxy-5-methyl-benzol.
   farbloser Feststoff;
   mp: 196°C; MS (DCI): 290 (M⁺)
9 c) Die Dicarbonsäure aus 9 b) wurde gemäß Variante 1 A ins Diguanidid überführt und als Hydrochlorid isoliert.
   farbloser Feststoff;
   mp: 279°C; MS (NBA):373 (M+1)⁺

### Beispiel 10: 1,2-Bis-[3-(E-2-methyl-propensäureguanidid)]-4-methyl-benzol-dihydrochlorid

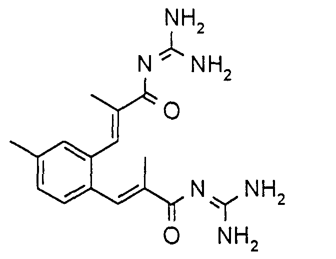

10 a, b) 4-Methyl-phthalsäurediester wurde gemäß Standardmethode (z. B. Reduktion mit Lithiumaluminiumhydrid) in den Dialkohol 10 a) überführt. Anschließend wurde der Alkohol unter Standardbedingungen zum Dialdehyd 10 b) oxidiert (z. B. Swern Oxidation).
   Dialkohol 10 a): farbloses Öl; MS (DCI): 153 (M+1⁺) und 135 (M+1-H₂O).
   Dialdehyd 10 b): dunkles Öl, MS (CI): 149 (M+1⁺)
10 c) 1 eq. 2-Phosphonopropionsäuretriethylester wurde bei 0°C mit 1 eq. n-Butyllithium in Hexan deprotoniert und anschließend bei RT mit 0.5 eq. 4-Methyl-1,2-phthaldialdehyd 10 b) versetzt. Nach vollständiger Abreaktion des Dialdehyds wurde mit Wasser aufgearbeitet und dreimal mit Toluol ausgeschüttelt. Nach Trocknen der vereinigten organischen Phasen über Magnesiumsulfat wurde das Lösungsmittel im Vakuum entfernt und das verbleibende Rohprodukt chromatographisch an Kieselgel mit EE/HEP-Gemischen als Eluens getrennt. Man isolierte: 4-Methyl-1,2-di-[3-(E-2-methyl-propensäureethylester)]-benzol.
   farbloses Öl; MS (CI):317 (M+1 ⁺)
10 d) Der Ester aus 10 c) wurde gemäß einer Standardmethode (Natrium-hydroxid in Methanol) verseift. Man isolierte: 4-Methyl-1,2-di-[3-(E-2-methyl-propensäure )]-benzol.
   farbloser Feststoff;
   mp: 194 - 198°C; MS (CI):260 (M⁺)
10 e) Die Dicarbonsäure aus 10 d) wurde gemäß Variante 1 A ins Diguanidid-dihydrochlorid überführt.
   farbloser Feststoff;
   mp: 190°C; MS (ES):342 (M+1)⁺

### Beispiel 11: 1,2-Bis-(3-(E-2-methyl-propensäureguanidld)]-4,5-dichlor-benzol-dihydrochlorid

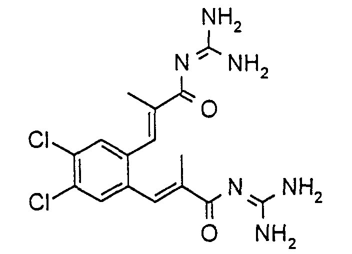

11 a, b) 4,5-Dichlor-phthalsäurediethylester wurde gemäß Standardmethode (z. B. Reduktion mit Lithiumaluminiumhydrid) in den Dialkohol 11 a) überführt. Anschließend wurde der Alkohol unter Standardbedingungen zum Dialdehyd 11 b) oxidiert (z. B. Swern Oxidation).
   Dialkohol 11 a): farbloser Feststoff, mp 147°C; MS (CI): 207 (M+1⁺)
   Dialdehyd 11 b): amorpher Feststoff, MS (CI): 203 (M+1⁺)
11 c) 1 eq. 2-Phosphonopropionsäuretriethylester wurde bei 0°C mit 1 eq. n-Butyllithium in Hexan deprotoniert und anschließend bei RT mit 0.5 eq. 4,5-Dichlor-1,2-phthaldialdehyd 11 b) versetzt. Nach vollständiger Abreaktion des Dialdehyds wurde mit Wasser aufgearbeitet und dreimal mit Toluol ausgeschüttelt. Nach Trocknen der vereinigten organischen Phasen über Magnesiumsulfat wurde das Lösungsmittel im Vakuum entfernt und das verbleibende Rohprodukt chromatographisch an Kieselgel mit EE/HEP-Gemischen als Eluens getrennt. Man isolierte: 4,5-Dichlor-1,2-di-[3-(E-2-methyl-propensäureethylester)]-benzol.
   farbloser Feststoff;
   mp: >230°C; MS (Cl):371 (M+1⁺)
11 d) Der Diester aus 11 c) wurde gemäß Variante 1 B ins Diguanidid-dihydrochlorid überführt.
   farbloser Feststoff;
   mp: >220°C; MS (ES):397 (M+1)⁺

### Beispiel 12: 1,3-Bis-(3-(E-propensäureguanidid)]benzol-dihydrochlorid

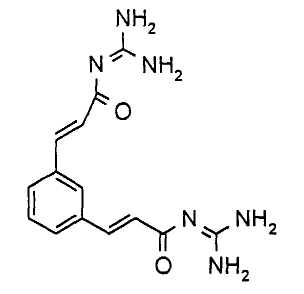

12 a) 1 eq. Triethylphosphonacetat wurde bei 0°C mit 1 eq. n-Butyllithium in Hexan deprotoniert und anschließend bei RT mit 0.5 eq. 1,3-lsophthaldialdehyd versetzt. Nach vollständiger Abreaktion des Dialdehyds wurde mit Wasser aufgearbeitet und dreimal mit Toluol ausgeschüttelt. Nach Trocknen der vereinigten organischen Phasen über Magnesiumsulfat wurde das Lösungsmittel im Vakuum entfernt und das verbleibende Rohprodukt chromatographisch an Kieselgel mit EE/HEP-Gemischen als Eluens getrennt. Man isolierte: 1,3-Di-[3-(E-propensäureethylester)]-benzol.
   farbloses Öl; MS (Cl):275 (M+1⁺)
12 b) Der Diester aus 12 a) wurde gemäß einer Standardmethode (Natrium-hydroxid in Methanol) verseift. Man isolierte: 1,3-Di-[3-(E-propensäure)]-benzol. farbloser Feststoff;
   mp: >200°C; MS (DCI): 217 (M-1⁺)
12 c) Die Dicarbonsäure aus 12 b) wurde gemäß Variante 1 A ins Diguanidid-dihydrochlorid überführt.
   farbloser Feststoff;
   mp: 296°C; MS (ES):301 (M+1)⁺

### Beispiel 13: 1,2-Bis-(3-(E-2-methy(-propensäureguanidid)]-4-brom-benzol-dihydrochlorid

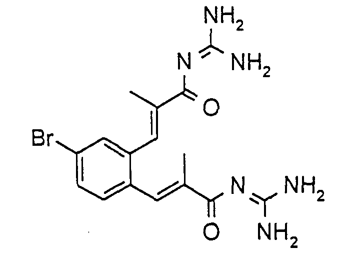

13 a, b) 4-Brom-phthalsäuredimethylester wurde gemäß Standardmethode (z. B. Reduktion mit Lithiumaluminiumhydrid) in den Dialkohol 13 a) überführt. Anschließend wurde der Alkohol unter Standardbedingungen zum Dialdehyd 13 b) oxidiert (z. B. Swern Oxidation).
   Dialkohol 13 a): farbloses Öl; MS (DCI): 217 (M+1⁺) und 199 (M+1-H₂O).
   Dialdehyd 13 b): amorpher Feststoff, MS (CI): 213 (M+1⁺)
13 c) 1 eq. 2-Phosphonopropionsäuretriethylester wurde bei 0°C mit 1 eq. n-Butyllithium in Hexan deprotoniert und anschließend bei RT mit 0.5 eq. 4-Brom-1,2-phthaldialdehyd 13 b) versetzt. Nach vollständiger Abreaktion des Dialdehyds wurde mit Wasser aufgearbeitet und dreimal mit Toluol ausgeschüttelt. Nach Trocknen der vereinigten organischen Phasen über Magnesiumsulfat wurde das Lösungsmittel im Vakuum entfernt und das verbleibende Rohprodukt chromatographisch an Kieselgel mit EE/HEP-Gemischen als Eluens getrennt. Man isolierte: 4-Brom-1,2-di-[3-(E-2-methyl-propensäureethylester)]-benzol.
   farbloses Öl; MS (CI):381 (M+1⁺)
13 d) Der Ester aus 13 c) wurde gemäß einer Standardmethode (Natrium-hydroxid in Methanol) verseift. Man isolierte: 4-Brom-1,2-di-[3-(E-2-methyl-propensäure)]-benzol.
   farbloser amorpher Feststoff; MS (ES):325 (M+1⁺)
13 e) Die Dicarbonsäure aus 13 e) wurde gemäß Variante 1 A ins Diguanidid-dihydrochlorid überführt.
   farbloser Feststoff;
   mp: 240°C; MS (FAB):407 (M+1⁺)

### Beispiel 14: 1,2-Bis-[3-(E-2-methyl-propensäureguanidid)]-4-(4-methoxyphenoxy)-benzol-dihydrochlorid

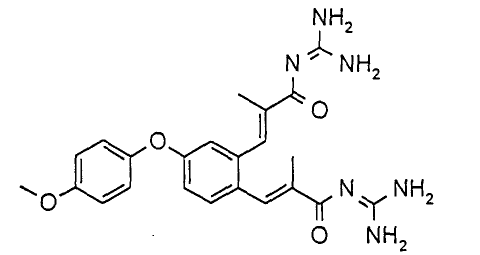

14 a) 4-Nitro-Phthalsäuredimethylester wurde in Analogie zu einem literaturbekannten Verfahren (J. Org. Chem. Vol. 42, No. 21, 1977, 3419-3425) in DMF mit 4-Methoxyphenolat Natriumsalz zum 4-(4-Methoxyphenoxy)phthalsäuredimethylester umgesetzt. Nach Standardaufarbeitung und Chromatographie mit Hexan/EE isolierte man den Diester als bräunliches Öl.
   MS (CI): 316 (M⁺); 317 (M+1⁺).
14 b, c) Der Diester 14 a) wurde gemäß Standardmethode (z. B. Reduktion mit Lithiumaluminiumhydrid) in den Dialkohol 14 b) überführt. Anschließend wurde der Alkohol unter Standardbedingungen zum Dialdehyd 14 c) oxidiert (z. B. Swern Oxidation).
   Dialkohol 14 b): bräunliches Öl; MS (CI): 260 (M⁺).
   Dialdehyd 14 c): bräunliches Öl, MS (CI): 257 (M+1⁺).
14 d) 1 eq. 2-Phosphonopropionsäuretriethylester wurde bei 0°C mit 1 eq. n-Butyllithium in Hexan deprotoniert und anschließend bei RT mit 0.5 eq. 1,2-Phthaldialdehyd 14 c) versetzt. Nach vollständiger Abreaktion des Dialdehyds wurde mit Wasser aufgearbeitet und dreimal mit Toluol ausgeschüttelt. Nach Trocknen der vereinigten organischen Phasen über Magnesiumsulfat wurde das Lösungsmittel im Vakuum entfernt und das verbleibende Rohprodukt chromatographisch an Kieselgel mit EE/HEP-Gemischen als Eluens getrennt. Man isolierte: 1,2-Di-[3-(E-2-methyl-propensäureethylester)]-4-(4-Methoxyphenoxy)benzol.
   leicht bräunliches Öl; MS (NBA):424 (M⁺)
14 e) Der Diester aus 14 d) wurde gemäß einer Standardmethode (Natrium-hydroxid in Methanol) verseift. Man isolierte: 1,2-Di-[3-(E-2-methyl-propensäure)]-4-(4-Methoxyphenoxy )benzol.
   hellgelber Feststoff;
   mp: 112°C; MS (ES):368(M⁺)
14 f) Die Dicarbonsäure aus 14 e) wurde gemäß Variante 1 A ins Diguanidid-dihydrochlorid überführt.
   farbloser Feststoff;
   mp: 212°C; MS (ES):451 (M+1)⁺

### Beispiel 15: 1,2-Bis-[3-(E-2-methyl-propensäureguanidid)]-4-(4-methyl-phenoxy)-benzol-dihydrochlorid

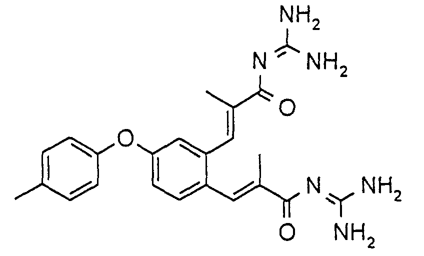

15 a) 4-Nitro-Phthalsäuredimethylester wurde in Analogie zu einem literaturbekannten Verfahren (J. Org. Chem. Vol. 42, No. 21, 1977, 3419-3425) in DMF mit 4-Methylphenolat Natriumsalz zum 4-(4-Methyl-phenoxy)phthalsäuredimethylester umgesetzt. Nach Standardaufarbeitung und Chromatographie mit Hexan/EE isolierte man den Diester als gelbliches Öl.
   MS (CI): 301 (M+1⁺).
15 b, c) Der Diester 15 a) wurde gemäß Standardmethode (z. B. Reduktion mit Lithiumaluminiumhydrid) in den Dialkohol 15 b) überführt. Anschließend wurde der Alkohol unter Standardbedingungen zum Dialdehyd 15 c) oxidiert (z. B. Swern Oxidation).
   Dialkohol 15 b): gelbliches Öl; MS (CI): 244 (M⁺), 245 (M+1 ⁺).
   Dialdehyd 15 c): bräunliches Öl, MS (CI): 241 (M+1 ⁺).
15 d) 1 eq. 2-Phosphonopropionsäuretriethylester wurde bei 0°C mit 1 eq. n-Butyllithium in Hexan deprotoniert und anschließend bei RT mit 0.5 eq. 1,2-Phthaldialdehyd 15 c) versetzt. Nach vollständiger Abreaktion des Dialdehyds wurde mit Wasser aufgearbeitet und dreimal mit Toluol ausgeschüttelt. Nach Trocknen der vereinigten organischen Phasen über Magnesiumsulfat wurde das Lösungsmittel im Vakuum entfernt und das verbleibende Rohprodukt chromatographisch an Kieselgel mit EE/HEP-Gemischen als Eluens getrennt. Man isolierte: 1,2-Di-[3-(E-2-methyl-propensäureethylester)]-4-(4-methyl-phenoxy)benzol.
   leicht bräunliches Öl; MS (NBA):408 (M⁺), 409 (M+1⁺)
15 e) Der Diester aus 15 d) wurde gemäß einer Standardmethode (Natrium-hydroxid in Methanol) verseift. Man isolierte: 1,2-Di-[3-(E-2-methyl-propensäure)]-4-(4-methyl-phenoxy)benzol.
   farbloser Feststoff;
   mp: 185°C; MS (NBA):352 (M⁺)
15 f) Die Dicarbonsäure aus 15 e) wurde gemäß Variante 1 A ins Diguanidid-dihydrochlorid überführt.
   farbloser Feststoff;
   mp: 186°C; MS (ES):435 (M+1)⁺

### Beispiel 16: 1,3-Bis-[3-(E-2-methyl-propensäureguanidid)]-5-methoxy-benzol-hydrochlorid

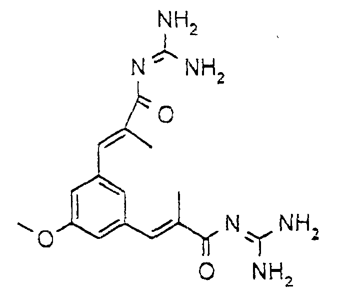

16 a) 1 eq. 2-Phosphonopropionsäuretriethylester wurde bei 0°C mit 1 eq. n-Butyllithium in Hexan deprotoniert und anschiießend bei RT mit 0.5 eq. 5-Methoxy-1,3-isophthaldialdehyd versetzt. Nach vollständiger Abreaktion des Dialdehyds wurde mit Wasser aufgearbeitet und dreimal mit Toluol ausgeschüttelt. Nach Trocknen der vereinigten organischen Phasen über Magnesiumsulfat wurde das Lösungsmittel im Vakuum entfernt und das verbleibende Rohprodukt chromatographisch an Kieselgel mit EE/HEP-Gemischen als Eluens getrennt. Man isolierte: 1,3-Di-[3-(E-2-methyl-propensäureethylester)]-5-methoxy-benzol.
   farbloses Öl; MS (CI):333 (M+1⁺)
16 b) Der Diester aus 16 a) wurde gemäß einer Standardmethode (Natrium-hydroxid in Methanol) verseift. Man isolierte : 1,3-Di-[3-(E-2-methyl-propensäure)]-5-methoxy-benzol.
   farbloser Feststoff;
   mp: >200°C; MS (DCI): 276 (M⁺)
16 c) Die Dicarbonsäure aus 16 b) wurde gemäß Variante 1 A ins Diguanidid überführt und als Hydrochlorid isoliert.
   farbloser Feststoff;
   mp: 124°C; MS (NBA):359 (M+1)⁺

### Beispiel 17: 1,3-Bis-[3-(E-2-methyl-propensäureguanidid)]-5-tert.-butyl-benzol-hydrochlorid

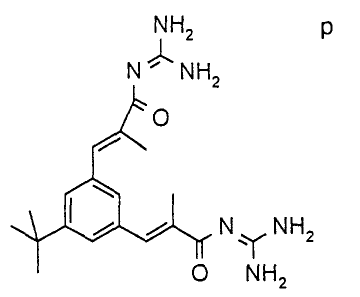

17 a) 1 eq. 2-Phosphonopropionsäuretriethylester wurde bei 0°C mit 1 eq. n-Butyllithium in Hexan deprotoniert und anschließend bei RT mit 0.5 eq. 5-tert.-butyl-1,3-isophthaldialdehyd versetzt. Nach vollständiger Abreaktion des Dialdehyds wurde mit Wasser aufgearbeitet und dreimal mit Toluol ausgeschüttelt. Nach Trocknen der vereinigten organischen Phasen über Magnesiumsulfat wurde das Lösungsmittel im Vakuum entfernt und das verbleibende Rohprodukt chromatographisch an Kieselgel mit EE/HEP-Gemischen als Eluens getrennt. Man isolierte: 1,3-Di-[3-(E-2-methyl-propensäureethylester)]-5-methoxy-benzol.
   farbloses Öl; MS (CI):359 (M+1⁺)
17 b) Der Diester aus 17 a) wurde gemäß einer Standardmethode (Natrium-hydroxid in Methanol) verseift. Man isolierte: 1,3-Di-[3-(E-2-methyl-propensäure)]-5-tert.-butyl-benzol.
   farbloser Feststoff;
   mp: >200°C; MS (DCI): 302 (M⁺)
17 c) Die Dicarbonsäure aus 17 b) wurde gemäß Variante 1 A ins Diguanidid überführt und als Hydrochlorid isoliert.
   farbloser Feststoff;
   mp:115°C; MS (NBA):385 (M+1)⁺

### Beispiel 18:1,4-Bis-[3-(E-2-methyl-propensäureguanidid)]-2,5-dichlor-benzol-dihydrochlorid

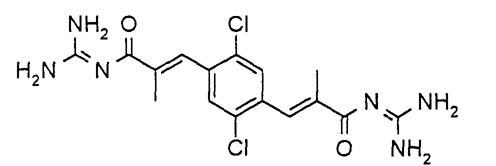

18 a) 1 eq. 2-Phosphonopropionsäuretriethylester wurde bei 0°C mit 1 eq. n-Butyllithium in Hexan deprotoniert und anschließend bei RT mit 0.5 eq. 1,4-(2,5-Dichlor)-terephthaldialdehyd versetzt. Nach vollständiger Abreaktion des Dialdehyds wurde mit Wasser aufgearbeitet und dreimal mit Toluol ausgeschüttelt. Nach Trocknen der vereinigten organischen Phasen über Magnesiumsulfat wurde das Lösungsmittel im Vakuum entfernt und das verbleibende Rohprodukt chromatographisch an Kieselgel mit EE/HEP-Gemischen als Eluens getrennt. Man isolierte: 1,4-Di-[3-(E-2-methyl-propensäureethylester)]-2,5-dichlor-benzol.
   farbloser Feststoff;
   mp: amorph; MS (DCI):371 (M+1⁺)
18 b) Der Ester aus 18 a) wurde gemäß einer Standardmethode (Natrium-hydroxid in Methanol) verseift. Man isolierte: 1,4-Di-[3-(E-2-methyl-propensäure)]-2,5-dichlor-benzol.
   farbloser Feststoff; MS (DCI): 315 (M+1⁺)
18 c) Die Dicarbonsäure aus 18 b) wurde gemäß Variante 1 A ins Diguanidid-dihydrochlorid überführt.
   gelber Feststoff;
   mp: >200°C; MS (DCI):397 (M+1)⁺

### Beispiel 19: 1,2-Bis-[3-(E-2-methyl-propensäureguanidid)]-4-(phenoxy)-benzol-dihydrochlorid

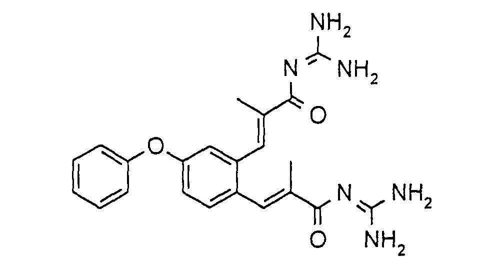

19 a) 4-Nitro-Phthalsäuredimethylester wurde in Analogie zu einem literaturbekannten Verfahren (J. Org. Chem. Vol. 42, No. 21, 1977, 3419-3425) in DMF mit Phenolat Natriumsalz zum 4-Phenoxy-phthalsäuredimethylester umgesetzt. Nach Standardaufarbeitung und Chromatographie mit Hexan/EE isolierte man den Diester als gelbliches Öl.
   MS (CI):287 (M+1⁺).
19 b, c) Der Diester 19 a) wurde gemäß Standardmethode (z. B. Reduktion mit Lithiumaluminiumhydrid) in den Dialkohol 19 b) überführt. Anschließend wurde der Alkohol unter Standardbedingungen zum Dialdehyd 19 c) oxidiert (z. B. Swern Oxidation).
   Dialkohol 19 b): gelbliches Öl; MS (CI): 230 (M⁺), 231 (M+1⁺).
   Dialdehyd 19 c): bräunliches Öl, MS (CI): 227 (M+1⁺).
19 d) 1 eq. 2-Phosphonopropionsäuretriethylester wurde bei 0°C mit 1 eq. n-Butyllithium in Hexan deprotoniert und anschließend bei RT mit 0.5 eq. 1,2-Phthaldialdehyd 19 c) versetzt. Nach vollständiger Abreaktion des Dialdehyds wurde mit Wasser aufgearbeitet und dreimal mit Toluol ausgeschüttelt. Nach Trocknen der vereinigten organischen Phasen über Magnesiumsulfat wurde das Lösungsmittel im Vakuum entfernt und das verbleibende Rohprodukt chromatographisch an Kieselgel mit EE/HEP-Gemischen als Eluens getrennt. Man isolierte: 1,2-Di-[3-(E-2-methyl-propensäureethylester)]-4-(phenoxy)benzol.
   leicht bräunliches Öl; MS (NBA):394 (M⁺), 395 (M+1⁺)
19 e) Der Diester aus 19 d) wurde gemäß einer Standardmethode (Natrium-hydroxid in Methanol) verseift. Man isolierte: 1,2-Di-[3-(E-2-methyl-propensäure)]-4-(phenoxy)benzol.
   farbloser Feststoff;
   mp: 160°C; MS (NBA):338 (M⁺)
19 f) Die Dicarbonsäure aus 19 e) wurde gemäß Variante 1 A ins Diguanidid-dihydrochlorid überführt.
   farbloser Feststoff;
   mp: 170°C; MS (ES): 421 (M+1)⁺

### Beispiel 20: 1,2-Bis-[3-(E-2-methyl-propensäureguanidid)]-4-(methoxy)-benzol-dihvdrochlorid

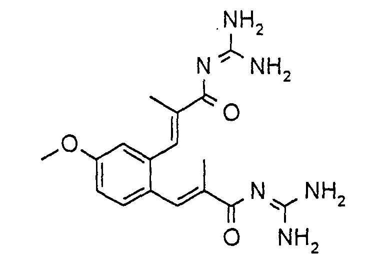

In Analogie zu Beispiel 19 wurde Beispiel 20 durch nucleophile aromatische Substitution des 4-Nitrophthalsäureesters mit Natriummethylat, anschließende Reduktion/Oxidation/ Olefinierungsreaktion, Verseifung und Guanidierung der Disäure dargestellt.
20 a) 4-Methoxy4-Methoxy-phthalsäurediester MS (Cl): 225 (M+1⁺).
   Dialkohol 20 b): gelb-bräunliches Öl; MS (CI): 169 (M+1 ⁺).
   Dialdehyd 20 c): dunkles Öl, MS (CI): 165 (M+1 ⁺).
   Diester 20 d): dunkles Öl; MS (NBA): 333 (M+1⁺)
20 e) 1,2-Di-[3-(E-2-methyl-propensäure)]-4-(methoxy)benzol
   farbloser Feststoff;
   mp: >200°C; MS (NBA):276 (M⁺)
20 f) Die Dicarbonsäure aus 20 e) wurde gemäß Variante 1 A ins Diguanidid-dihydrochlorid überführt.
   farbloser Feststoff;
   mp: 170°C; MS (ES):359 (M+1)⁺

### Beispiel 21: 1,2-Bis-(3-(E-2-methyl-propensäureguanidid)]-4-(ethoxy)-benzol-dihydrochlorid

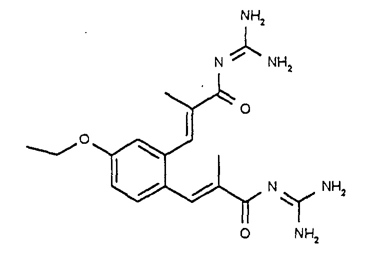

21 a) 4-Ethoxy-phthalsäurediethylester wurde durch Ethylierung der 4-Hydroxyphthalsäure mit 3.5 Äquivalenten Ethyliodid und 3.1 Äquivalenten Kaliumcarbonat in DMF bei 70°C dargestellt. Anschließend wurde via Reduktion/Oxidation/ Olefinierungsreaktion der Diester dargestellt, der gemäß Variante 1 B ins Diguanidid 21 überführt wurde.
21 a) 4-Ethoxy-phthalsäurediethylester; dunkles Öl; MS (Cl): 267 (M+1 ⁺). Dialkohol 21 b): gelb-bräunliches Öl; MS (Cl): 183 (M+1 ⁺).
   Dialdehyd 21 c): dunkles Öl, MS (Cl): 179 (M+1 ⁺).
   Diester 21 d): gelbliches Öl; MS (NBA): 347 (M+1⁺)
21 f) Der Diester 20 d) wurde gemäß Variante 1 B ins Diguanidid-dihydrochlorid überführt.
   farbloser Feststoff.
   mp: 245°C; MS (ES):373 (M+1)⁺

### Beispiel 22: 1,2-Bis-[3-(E-2-methyl-propensäureguanidid)]-4-(3-pyridyloxy)-benzol-dihydrochlorid

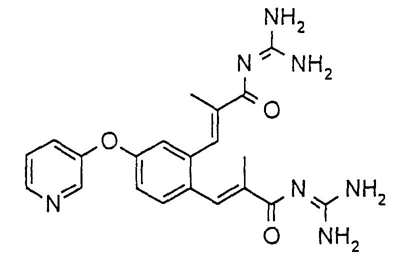

22 a) 4-Nitro-Phthalsäuredimethylester wurde in Analogie zu einem literaturbekannten Verfahren (J. Org. Chem. Vol. 42, No. 21, 1977, 3419-3425) in DMF mit 3-Hydroxypyridin Natriumsalz zum 4-(3-pyridyloxy)-phthalsäuredimethylester umgesetzt. Nach Standardaufarbeitung und Chromatographie mit Hexan/EE isolierte man den Diester als gelbliches Öl.
   MS: 288 (M+1⁺).
22 b, c) Der Diester 22 a) wurde gemäß Standardmethode (z. B. Reduktion mit Lithiumaluminiumhydrid) in den Dialkohol 22 b) überführt. Anschließend wurde der Alkohol unter Standardbedingungen zum Dialdehyd 22 c) oxidiert (z. B. Swern Oxidation).
   Dialkohol 22 b): gelbliches Öl; MS: 232 (M+1⁺).
   Dialdehyd 22 c): gelb-bräunliches Öl, MS: 228 (M+1⁺).
22 d) 1 eq. 2-Phosphonopropionsäuretriethylester wurde bei 0°C mit 1 eq. n-Butyllithium in Hexan deprotoniert und anschließend bei RT mit 0.5 eq. 1,2-Phthaldialdehyd 22 c) versetzt. Nach vollständiger Abreaktion des Dialdehyds wurde mit Wasser aufgearbeitet und dreimal mit Toluol ausgeschüttelt. Nach Trocknen der vereinigten organischen Phasen über Magnesiumsulfat wurde das Lösungsmittel im Vakuum entfernt und das verbleibende Rohprodukt chromatographisch an Kieselgel mit EE/HEP-Gemischen als Eluens getrennt. Man isolierte: 1,2-Di-[3-(E-2-methyl-propensäureethylester)]-4-(3-pyridyloxy)-benzol.
   leicht bräunliches Öl; MS: 396 (M+1⁺)
22 e) Der Diester aus 22 d) wurde gemäß einer Standardmethode (Natrium-hydroxid in Methanol) verseift. Man isolierte : 1,2-Di-[3-(E-2-methyl-propensäure)]-4-(3-pyridyloxy)-benzol.
   farbloser Feststoff;
   mp: 210°C; MS: 339 (M⁺)
22 f) Die Dicarbonsäure aus 22 e) wurde gemäß Variante 1 A ins Diguanidid-dihydrochlorid überführt.
   farbloser Feststoff;
   mp: 176°C; MS: 422 (M+1)⁺

### Beispiel 23: 1,2-Bis-[3-(E-2-methyl-propensäureguanidid)]-4-[4-(2-dimethylaminoethylen)-phenoxy]-benzol-dihydrochlorid

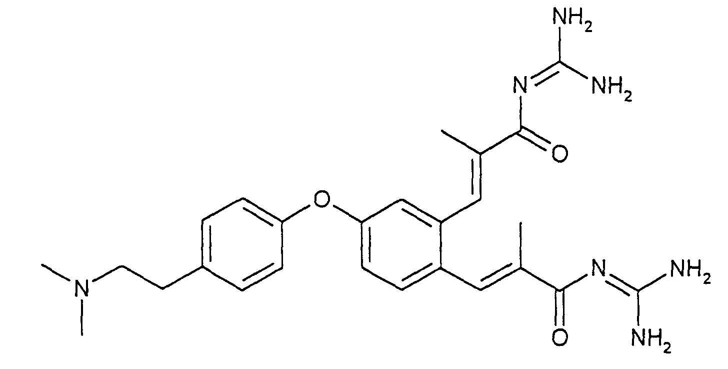

23 a) 4-Nitro-Phthalsäuredimethylester wurde in Analogie zu einem literaturbekannten Verfahren (J. Org. Chem. Vol. 42, No. 21, 1977, 3419-3425) in DMF mit 4-(2-dimethylaminoethylen)-phenol Natriumsalz zum 4-[4-(2-dimethylaminoethylen)-phenoxy]-phthalsäuredimethylester umgesetzt. Nach Standardaufarbeitung und Chromatographie mit Hexan/EE isolierte man den Diester.
23 b, c) Der Diester 23 a) wurde gemäß Standardmethode (Reduktion mit Lithiumaluminiumhydrid) in den Dialkohol 23 b) überführt. Anschließend wurde der Alkohol gemäß Dess-Martin (siehe Dess-Martin-Oxidation; JOC, 1994, 59, 7549 - 7552) zum Dialdehyd 23 c) oxidiert.
   Dialkohol 23 b): gelb-braunes Öl; MS: 340 (M+1 ⁺).
   Dialdehyd 23 c): gelbes Öl, MS: 269 (M+1⁺).
23 d) 1 eq. 2-Phosphonopropionsäuretriethylester wurde bei 0°C mit 1 eq. n-Butyllithium in Hexan deprotoniert und anschließend bei RT mit 0.5 eq. 1,2-Phthaldialdehyd 23 c) versetzt. Nach vollständiger Abreaktion des Dialdehyds wurde mit Wasser aufgearbeitet und dreimal mit Toluol ausgeschüttelt. Nach Trocknen der vereinigten organischen Phasen über Magnesiumsulfat wurde das Lösungsmittel im Vakuum entfernt und das verbleibende Rohprodukt chromatographisch an Kieselgel mit EE/HEP-Gemischen als Eluens getrennt. Man isolierte : 1,2-Bis-[3-(E-2-methyl-propensäureethylester)]-4-[4-(2-dimethylaminoethylen)-phenoxy]-benzol.
   Gelbliches Öl; MS: 466 (M+1⁺)
23 e) Der Diester aus 23 d) wurde gemäß einer Standardmethode (Natriumhydroxid in Methanol) verseift. Man isolierte: 1,2-Bis-[3-(E-2-methyl-propensäure)]-4-[4-(2-dimethylaminoethylen)-phenoxy]-benzol.
   farbloser Feststoff;
   mp: >220°C; MS: 409 (M⁺)
23 f) Die Dicarbonsäure aus 23 e) wurde gemäß Variante 1 A ins Diguanidid-dihydrochlorid überführt.
   MS: 410 (M+1)⁺

### Beispiel 24 und 25: 1,2-Bis-[3-(E-2-methyl-propensäureguanidid)]-4-[4-methoxybenzyloxy]-benzol-dihydrochlorid und 1,2-Bis-[3-(E-2-methyl-propensäureguanidid)]-4-hydroxy-benzol-dihydrochlorid

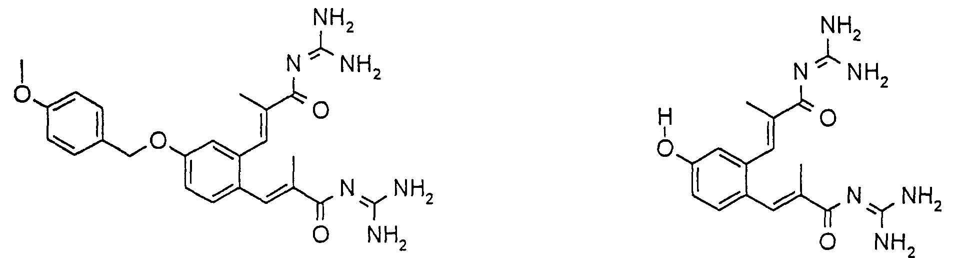

24 a) 1 eq. 4-Hydroxyphthalsäuredimethylester, 1.1 eq. Kaliumcarbonat und 1.1 eq. 4-Methoxybenzylchlorid wurden in DMF bei RT gerührt. Nach 4 Tagen wurde gemäß Standardvorschrift aufgearbeitet. Man isolierte 4-(4-Methoxybenzyloxy)-phthalsäuredimethylester als farbloses Öl.
   MS: 331 (M+1⁺).
24 b, c) Der Diester 24 a) wurde gemäß Standardmethode (Reduktion mit Lithiumaluminiumhydrid) in den Dialkohol 24 b) überführt. Anschließend wurde der Alkohol gemäß Standardvorschrift (Swern-Oxidation) zum Dialdehyd 24 c) oxidiert.
   Dialkohol 24 b): amorpher Feststoff; MS: 275 (M+1⁺).
   Dialdehyd 24 c): gelbliches Öl, MS: 271 (M+1⁺).
24 d) 1 eq. 2-Phosphonopropionsäuretriethylester wurde bei 0°C mit 1 eq. n-Butyllithium in Hexan deprotoniert und anschließend bei RT mit 0.5 eq. 1,2-Phthaldialdehyd 24 c) versetzt. Nach vollständiger Abreaktion des Dialdehyds wurde mit Wasser aufgearbeitet und dreimal mit Toluol ausgeschüttelt. Nach Trocknen der vereinigten organischen Phasen über Magnesiumsulfat wurde das Lösungsmittel im Vakuum entfernt und das verbleibende Rohprodukt chromatographisch an Kieselgel mit EE/HEP-Gemischen als Eluens getrennt. Man isolierte : 1,2-Bis-[3-(E-2-methyl-propensäuremethylester)]-4-[4-methoxybenzyloxy]-benzol, als gelbliches Öl.
   MS: 439 (M+1⁺)
24 e) Der Diester aus 24 d) wurde gemäß einer Standardmethode (Natrium-hydroxid in Methanol) verseift. Man isolierte: 1,2-Bis-[3-(E-2-methyl-propensäure)]-4-[4-methoxybenzyloxy]-benzol, farbloser Feststoff.
   mp: 206 - 220°C; MS: 382 (M⁺)
24 f) Die Dicarbonsäure aus 24 e) wurde gemäß Variante 1 A ins Diguanidid-dihydrochlorid überführt.
   mp: 210°C; MS: 465 (M+1)⁺

### Beispiel 25: 1,2-Bis-[3-(E-2-methyl-propensäureguanidid)]-4-hydroxy-benzol-dihydrochlorid

Die Dicarbonsäure aus 24 e) wurde gemäß Variante 1 A zum Diguanidid-dihydrochlorid umgesetzt. Außer Beispiel 24 wurde das Produkt 25 isoliert.
MS: 345 (M+1)⁺

### Pharmakologische Daten:

### Inhibitoren des Na⁺/H⁺-Exchangers von Kaninchenerythozyten (Subtyp 1; NHE-1):

Weiße Neuseeland-Kaninchen (Ivanovas) erhielten eine Standard-Diät mit 2% Cholesterin für sechs Wochen, um den Na⁺/H⁺-Austausch zu aktivieren und so den Na⁺-Influx in die Erythrozyten via Na⁺/H⁺-Austausch flammenphotometrisch bestimmen zu können. Das Blut wurde den Ohrarterien entnommen und durch 25 IE/ml Kalium-Heparin ungerinnbar gemacht. Ein Teil jeder Probe wurde zur Doppelbestimmung des Hämatokrits durch Zentrifugation benutzt. Aliquots von jeweils 100µl dienten zur Messung des Na⁺- Ausgangsgehalts der Erythrozyten.

Um den Amilorid-sensitiven Natrium-Influx zu bestimmen, wurden 100µl jeder Blutprobe in jeweils 5 ml eines hyperosmolaren Salz-Sucrose-Mediums (mmol/l: 140 NaCI, 3 KCI, 150 Sucrose, 0,1 Ouabain, 20 Tris-hydroxymethyl-aminomethan) bei pH 7,4 und 37°C inkubiert. Die Erythrozyten wurden danach dreimal mit eiskalter MgCl₂-Ouabain-Lösung (mmol/l: 112 MgCl₂, 0,1 Ouabain) gewaschen und in 2,0 ml destilliertem Wasser hämolysiert. Der intrazelluläre Natriumgehalt wurde flammenphotometrisch bestimmt.

Der Na⁺-Nettoinflux wurde aus der Differenz zwischen Natrium-Ausgangswerten und dem Natriumgehalt der Erythrozyten nach Inkubation errechnet. Der Amilorid-hemmbare Natrium-Influx ergab sich aus der Differenz des Natriumgehalts der Erythrozyten nach Inkubation mit und ohne Amilorid 3 x 10⁻⁴ mol/l. Auf diese Weise wurde auch bei den erfindungsgemäßen Verbindungen verfahren.

Ergebnisse zur Inhibition des Na⁺/H⁺-Exchangers (Subtyp 1; NHE-1):

| | |
|---|---|
| Beispiel ( siehe exp. Teil ) | IC₅₀ (µmol |
| 4 | 4 |

Die meisten der molekularbiologischen Techniken folgen Protokollen aus den Werken "Current Protocols in Molecular Biology (eds. Ausubel, F.M., Brent, R., Kingston, R.E., Moore, D.D., Seidman, J.G., Smith, J.A. und Struhl, K.; John Wiley & Sons)" bzw: "Molecular Cloning: A Laboratory Manual (Sambrock, J., Fritsch, E.F. und Maniatis, T.; Cold Spring Harbor Laboratory Press (1989))". Im Rahmen unserer Arbeiten wurden stabil transfizierte Zellinien erzeugt, die jeweils einen der folgenden NHE-Subtypen exprimieren: NHE1 des Menschen (Sardet et al.; Cell 56,271-280 (1989), NHE2 des Kaninchens (Tse et al.; J. Biol. Chem. 268, 11917 - 11924 (1993)) bzw. NHE3 der Ratte (Orlowski et al.; J. Biol. Chem. 267, 9331 - 9339 (1992)).

Die von Prof. Pouysségur erhaltenen cDNA-Klone der jeweiligen NHE-Subtypen wurden nach Anfügen geeigneter Linkersequenzen so in das Expressionsplasmid pMAMneo (erhältlich z. B. über CLONTECH, Heidelberg) einkloniert, daß die NHE1-Erkennungssequenz des Plasmids etwa 20 - 100 Basenpaare vor dem Startcodon des jeweiligen NHE-Subtyps liegt und die gesamte codierende Sequenz in dem Konstrukt vorhanden ist.

Mit der sog. "Calciumphosphat-Methode" (beschrieben in Kapitel 9.1 von "Current Protocols in Molecular Biology") wurde die NHE-defiziente Zellinie LAP1 (Franchi et al.; Proc. Natl. Acad. Sci. USA 83, 9388 - 9392 (1986)) mit den Plasmiden, die die jeweiligen codierenden Sequenzen der NHE-Subtypen erhalten, transfiziert. Nach Selektion auf transfizierte Zellen über Wachstum in G418-haltigem Medium (nur Zellen, die durch Transfektion ein neo-Gen erhalten haben, können unter diesen Bedingungen überleben) wurde auf eine funktionelle NHE-Expression selektioniert. Dazu wurde die von Sardet beschriebene "Acid Load"-Technik verwendet (Sardet et al.; Cell 56, 271 - 280 (1989)). Zellen, die einen funktionsfähigen NHE-Subtypen exprimieren, können auch bei Abwesenheit von CO₂ und HCO₃- die bei diesem Test vorgenommene Ansäuerung kompensieren, untransfizierte LAP1-Zellen dagegen nicht. Nach mehrmaliger Wiederholung der "Acid Load" Selektion wurden die überlebenden Zellen in Mikrotiterplatten so ausgesät, daß statistisch eine Zelle pro Well vorkommen sollte. Unter dem Mikroskop wurde nach etwa 10 Tagen überprüft, wie viele Kolonien pro Well wuchsen. Zellpopulationen aus Einzelkolonien wurden dann mit dem XTT-Proliferation Kit (Boehringer Mannheim) hinsichtlich ihrer Überlebensfähigkeit nach "Acid Load" untersucht. Die besten Zellinien wurden für die weiteren Tests verwendet und zur Vermeidung eines Verlustes der transfizierten Sequenz unter ständigem Selektionsdruck in G418-haltigem Medium kultiviert.

Zur Bestimmung von IC₅₀-Werten für die Hemmung der einzelnen NHE-Subtypen durch spezifische Substanzen wurde ein von S. Faber entwickelter Test (Faber et al.; Cell. Physiol. Biochem. 6, 39 - 49 (1996)), der auf der "Acid Load" Technik beruht, leicht abgewandelt.

In diesem Test wurde die Erholung des intrazellulären pHs (pHᵢ) nach einer Ansäuerung ermittelt, die bei funktionsfähigem NHE auch unter bicarbonatfreien Bedingungen einsetzt. Dazu wurde der pHᵢ mit dem pH-sensitiven Fluoreszenzfarbstoff BCECF (Calbiochem, eingesetzt wird die Vorstufe BCECF-AM) bestimmt. Die Zellen wurden zunächst mit BCECF beladen. Die BCECF-Fluoreszenz wurde in einem "Ratio Fluorescence Spectrometer" (Photon Technology International, South Brunswick, N.J., USA) bei Anregungswellenlängen von 505 und 440 nm und einer Emissionswellenlänge von 535 nm bestimmt und mittels Kalibrierungskurven in den pHᵢ umgerechnet. Abweichend von dem beschriebenen Protokoll wurden die Zellen bereits bei der BCECF-Beladung in NH₄Cl-Puffer (pH 7,4) inkubiert (NH₄Cl-Puffer: 115 mM NaCl, 20 mM NH₄Cl, 5 mM KCI, 1 mM CaCl₂, 1 mM MgSO₄, 20 mM Hepes, 5 mM Glucose, 1 mg/ml BSA; mit 1 M NaOH wird ein pH von 7,4 eingestellt). Die intrazelluläre Ansäuerung wurde durch Zugabe von 975 µl eines NH₄Cl-freien Puffers zu 25 µl Aliquots der in NH₄Cl-Puffer inkubierten Zellen induziert. Die nachfolgende Geschwindigkeit der pH-Erholung wurde bei NHE1 2 Minuten, bei NHE2 5 Minuten und bei NHE3 3 Minuten registriert. Für die Berechnung der inhibitorischen Potenz der getesteten Substanzen wurden die Zellen zunächst in Puffern untersucht, bei denen eine vollständige bzw. überhaupt keine pH-Erholung stattfand. Zur vollständigen pH-Erholung (100%) wurden die Zellen in Na⁺-haltigem Puffer inkubiert (133,8 mM NaCl, 4,7 mM KCI, 1,25 mM CaCl₂, 1,25 mM MgCl₂, 0,97 mM Na₂HPO₂, 0,23 mM NaH₂PO₄, 5 mM Hepes, 5 mM Glucose, mit 1 M NaOH wird ein pH von 7,0 eingestellt). Für die Bestimmung des 0%-Wertes wurden die Zellen in einem Na⁺-freien Puffer inkubiert (133,8 mM Cholinchlorid, 4,7 mM KCI, 1,25 mM CaCl₂, 1,25 mM MgCl₂, 0,97 mM K₂HPO₂, 0,23 mM KH₂PO₄, 5 mM Hepes, 5 mM Glucose, mit 1 M NaOH wird ein pH von 7,0 eingestellt). Die zu testenden Substanzen wurden in dem Na⁺-haltigem Puffer angesetzt. Die Erholung des intrazellulären pHs bei jeder getesteten Konzentration einer Substanz wurde in Prozent der maximalen Erholung ausgedrückt. Aus den Prozentwerten der pH-Erholung wurde mittels des Programms SigmaPlot der IC₅₀-Wert der jeweiligen Substanz für die einzelnen NHE-Subtypen berechnet.

| | IC₅₀; NHE-3 |
|---|---|
| Beispiel 1 | 0.7 |
| Beispiel 6 | 3.2 |
| Beispiel 9 | 1.75 |
| Beispiel 3 | 1.1 |

## Patentansprüche

1. Phenylsubstituierte Alkenylcarbonsäure-guanidide der Formel I 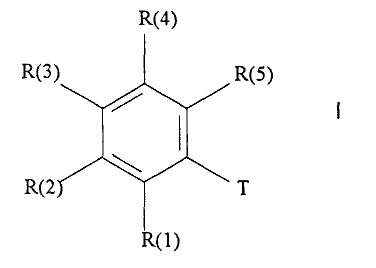 worin bedeuten:
T 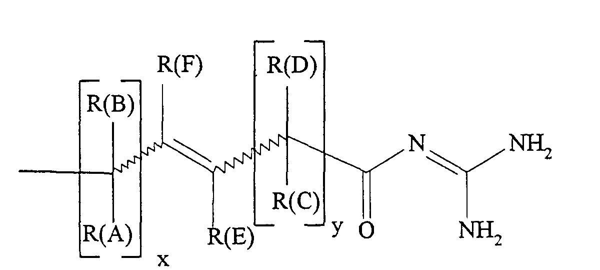
R(A) Wasserstoff, F, Cl, Br, I, CN, OH, OR(6), (C₁-C₄-Alkyl, Oᵣ(CH₂)ₐC_{b}F_{2b+1}, (C₃-C₈)-Cycloalkyl oder NR(7)R(8)
r Null oder 1;
a Null, 1, 2, 3 oder 4;
b 1, 2, 3 oder 4;
R(6) (C₁-C₄)-Alkyl, (C₁-C₄)-Perfluoralkyl, (C₃-C₆)-Alkenyl, (C₃-C₈)-Cycloalkyl, Phenyl oder Benzyl,
wobei der Phenylkern nicht substituiert oder substituiert ist mit 1 - 3 Substituenten ausgewählt aus der Gruppe bestehend aus F, Cl, CF₃, Methyl,
Methoxy und NR(9)R(10);
R(9) und R(10) H, (C₁-C₄)-Alkyl oder (C₁-C₄)-Perfluoralkyl;
R(7) und R(8) unabhängig voneinander wie R(6) definiert;
oder
R(7) und R(8) gemeinsam 4 oder 5 Methylengruppen, von denen eine CH₂-Gruppe durch Sauerstoff, Schwefel, NH, N-CH₃ oder N-Benzyl ersetzt sein kann;
R(B), R(C) und R(D) unabhängig wie R(A) definiert;
x Null, 1 oder 2;
y Null, 1 oder 2;
R(F) Wasserstoff, F, Cl, Br, I, CN, OR(12), (C₁-C₈)-Alkyl, Oₚ(CH₂)_{f}C_{g}F_{2g+1}, (C₃-C₈)-Cycloalkyl oder (C₁-C₉)-Heteroaryl;
p Null oder 1;
f Null, 1, 2, 3 oder 4;
g 1, 2, 3, 4, 5, 6, 7 oder 8;
R(12) (C₁-C₈)-Alkyl, (C₁-C₄)-Perfluoralkyl, (C₃-C₈)-Alkenyl, (C₃-C₈)-Cycloalkyl, Phenyl oder Benzyl,
wobei der Phenylkern nicht substituiert ist oder substituiert mit 1 - 3 Substituenten ausgewählt aus der Gruppe bestehend aus F, Cl, CF₃, Methyl, Methoxy und NR(13)R(14);
R(13) und R(14) H, (C₁-C₄)-Alkyl oder (C₁-C₄)-Perfluoralkyl;
R(E) unabhängig wie R(F) definiert;
R(1) unabhängig wie T definiert;
oder
R(1) Wasserstoff, -OₖCₘH₂ₘ₊₁, -Oₙ(CH₂)ₚC_{q}F_{2q+1}, F, Cl, Br, I, CN, -(C=O)-N=C(NH₂)₂, -SOᵣR(17), -SOᵣ₂NR(31)R(32); -Oᵤ(CH₂)ᵥC₆H₅, -Oᵤ₂-(C₁-C₉)-Heteroaryl oder-Sᵤ₂-(C₁-C₉)-Heteroaryl;
k Null oder 1;
m Null, 1, 2, 3, 4, 5, 6, 7 oder 8;
n Null oder 1;
p Null, 1, 2, 3 oder 4;
q 1, 2, 3, 4, 5, 6, 7 oder 8;
r Null, 1, 2;
r2 Null, 1, 2;
R(31) und R(32) unabhängig voneinander Wasserstoff, (C₁-C₈)-Alkyl oder (C₁-C₈)-Perfluoralkyl;
oder
R(31) und R(32) gemeinsam 4 oder 5 Methylengruppen, von denen eine CH₂-Gruppe durch Sauerstoff, S, NH, N-CH₃ oder N-Benzyl ersetzt sein kann;
R(17) (C₁-C₈)-Alkyl;
u Null oder 1;
u2 Null oder 1;
v Null, 1, 2, 3 oder 4;
wobei der Phenylkern unsubstituiert ist oder substituiert mit 1-3 Substituenten ausgewählt aus der Gruppe bestehend aus F, Cl, CF₃, Methyl, Methoxy, -(CH₂)_{w}NR(21)R(22), NR(18)R(19) und (C₁-C₉)-Heteroaryl;
R(18), R(19), R(21) und R(22) unabhängig voneinander (C₁-C₄)-Alkyl oder (C₁-C₄)-Perfluoralkyl;
w 1, 2, 3 oder 4;
wobei der Heterocyclus des (C₁-C₉)-Heteroaryl unsubstituiert oder substituiert ist mit 1 - 3 Substituenten ausgewählt aus der Gruppe bestehend aus F, Cl, CF₃, Methyl oder Methoxy;
R(2), R(3), R(4) und R(5) unabhängig voneinander wie R(1) definiert,
oder
R(1) und R(2) oder R(2) und R(3) jeweils gemeinsam -CH-CH=CH-CH-,
das nicht substituiert oder substituiert ist mit 1 - 3 Substituenten ausgewählt aus der Gruppe bestehend aus F, Cl, CF₃, Methyl, Methoxy, -(CH₂)_{w2}NR(24)R(25) und NR(26)R(27);
R(24), R(25), R(26) und R(27) H, (C₁-C₄)-Alkyl oder (C₁-C₄)-Perfluoralkyl;
w2 1, 2, 3 oder 4;
wobei der Rest T im Molekül mindestens zweimal, jedoch nur höchstens dreimal vorhanden ist;
sowie deren pharmazeutisch verträgliche Salze.

2. Verbindung der Formel I nach Anspruch 1, in der bedeuten:
T 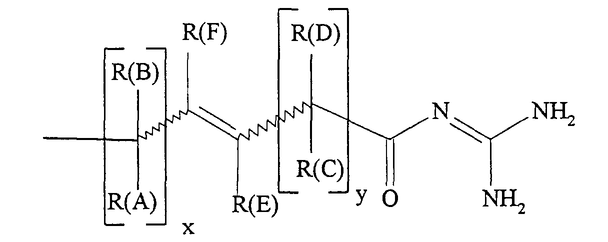
R(A) Wasserstoff, F, Cl, CN, OH, OR(6), (C₁-C₄)-Alkyl, Oᵣ(CH₂)ₐC_{b}F_{2b+1}, (C₃-C₈)-Cycloalkyl oder NR(7)R(8)
r Null oder 1;
a Null, 1 oder 2;
b 1, 2, 3 oder 4;
R(6) (C₁-C₄)-Alkyl, (C₁-C₄) -Perfluoralkyl, Phenyl oder Benzyl,
wobei der Phenylkern nicht substituiert ist oder substituiert mit 1 - 3 Substituenten ausgewählt aus der Gruppe bestehend aus F, Cl, CF₃, Methyl, Methoxy und NR(9)R(10);
R(9) und R(10) unabhängig voneinander H, CH₃ oder CF₃;
R(7) und R(8) unabhängig voneinander wie R(6) definiert;
oder
R(7) und R(8) gemeinsam 4 oder 5 Methylengruppen, von denen eine CH₂-Gruppe durch Sauerstoff, Schwefel, NH, N-CH₃ oder N-Benzyl ersetzt sein kann;
R(B), R(C), R(D) unabhängig wie R(A) definiert;
x Null oder 1;
y Null oder 1;
R(F) Wasserstoff, F, Cl, CN, OR(12), (C₁-C₄)-Alkyl, Oₚ(CH₂)_{f}C_{g}F_{2g+1}, (C₃-C₈)-Cycloalkyl oder (C₁-C₉)-Heteroaryl;
p Null oder 1;
f Null, 1 oder 2;
g 1, 2, 3 oder 4;
R(12) (C₁-C₄)-Alkyl, (C₁-C₄) -Perfluoralkyl, (C₃-C₈)-Cycloalkyl, Phenyl oder Benzyl,
wobei der Phenylkern unsubstituiert ist oder substituiert mit 1 - 3 Substituenten ausgewählt aus der Gruppe bestehend aus F, Cl, CF₃, Methyl, Methoxy und NR(13)R(14);
R(13) und R(14) unabhängig voneinander H, CH₃ oder CF₃;
R(E) unabhängig wie R(F) definiert;
R(1) unabhängig wie T definiert;
oder
R(1) Wasserstoff, -OₖCₘH₂ₘ₊₁, -OₙC_{q}F_{2q+1}, F, Cl, Br, l, CN, -(C=O)-N=C(NH₂)₂, -SOᵣR(17), -SOᵣ₂NR(31)R(32), -Oᵤ(CH₂)ᵥC₆H₅, -Oᵤ₂-(C1-C₉)-Heteroaryl oder -Sᵤ₂-(C₁-C₉)-Heteroaryl;
k Null oder 1;
m Null, 1, 2, 3 oder 4;
n Null oder 1;
q 1, 2, 3 oder 4;
r 2;
r2 Null, 1, 2;
R(31) und R(32) unabhängig voneinander Wasserstoff, (C₁-C₄)-Alkyl oder (C₁-C₄)-Perfluoralkyl;
oder
R(31) und R(32) gemeinsam 4 oder 5 Methylengruppen, von denen eine CH₂-Gruppe durch Sauerstoff, S, NH, N-CH₃ oder N-Benzyl ersetzt sein kann;
R(17) (C₁-C₄)-Alkyl;
u Null oder 1;
u2 Null oder 1;
v Null, 1 oder 2;
wobei der Phenylkern nicht substituiert oder substituiert ist mit 1-3 Substituenten ausgewählt aus der Gruppe bestehend aus F, Cl, CF₃, Methyl, Methoxy, -(CH₂)_{w}N(21)R(22), NR(18)R(19) oder C₁-(C₉)-Heteroaryl;
R(18), R(19), R(21) und R(22) H, (C₁-C₄)-Alkyl oder (C₁-C₄)-Perfluoralkyl;
w 1, 2, 3, 4;
wobei der Heterocyclus des (C₁-C₉)-Heteroaryl unsubstituiert oder substituiert ist mit 1 - 3 Substituenten ausgewählt aus der Gruppe bestehend aus F, Cl, CF₃, Methyl oder Methoxy;
R(2), R(3), R(4) und R(5) unabhängig voneinander wie R(1) definiert,
oder
R(1) und R(2) oder R(2) und R(3) jeweils gemeinsam -CH-CH=CH-CH-,
das nicht substituiert oder substituiert ist mit 1 - 3 Substituenten ausgewählt aus der Gruppe bestehend aus F, Cl, CF₃, Methyl, Methoxy, -(CH₂)_{w2}NR(24)R(25) und NR(26)R(27);
R(24), R(25), R(26) und R(27) H, CH₃ oder CF₃;
w2 1, 2, 3 oder 4;
wobei der Rest T im Molekül jedoch nur zweimal vorhanden ist.

3. Verbindung der Formel I nach Anspruch 1 oder 2, in der bedeuten:
T 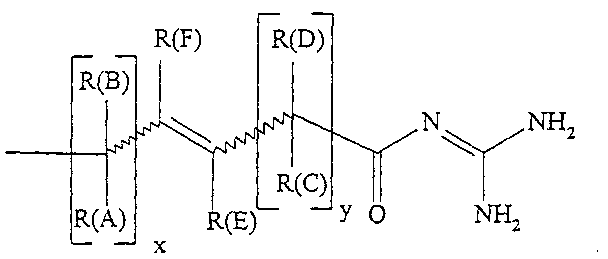
x Null;
y Null;
R(F) Wasserstoff, F, Cl, CN, OR(12), (C₁-C₄)-Alkyl, -OₚC_{g}F_{2g+1}, (C₃-C₈)-Cycloalkyl oder (C₁-C₉)-Heteroaryl;
p Null oder 1;
g 1, 2, 3 oder 4;
R(12) (C₁-C₄)-Alkyl, CF₃, (C₃-C₈)-Cycloalkyl, Phenyl oder Benzyl, wobei der Phenylkern jeweils nicht substituiert ist oder substituiert mit 1 - 3 Substituenten ausgewählt aus der Gruppe bestehend aus F, Cl, CF₃, Methyl, Methoxy und NR(13)R(14);
R(13) und R(14) H, CH₃ oder CF₃;
R(E) unabhängig wie R(F) definiert;
R(1) unabhängig wie T definiert;
oder
R(1) Wasserstoff, -OₖCₘH₂ₘ₊₁, -OₙC_{q}F_{2q+1}, F, Cl, CN, -(C=O)-N=C(NH₂)₂, -SO₂CH₃, -SO₂NR(31)R(32), -Oᵤ(CH₂)ᵥC₆H₅, -Oᵤ₂-(C₁-C₉)-Heteroaryl oder -Sᵤ₂-(C₁-C₉)-Heteroaryl;
k Null oder 1;
m Null, 1, 2, 3 oder 4;
n Null oder 1;
q 1, 2, 3 oder 4;
R(31) und R(32) unabhängig voneinander Wasserstoff oder (C₁-C₄)-Alkyl;
oder
R(31) und R(32) gemeinsam 4 oder 5 Methylengruppen, von denen eine CH₂-Gruppe durch Sauerstoff, S, NH, N-CH₃ oder N-Benzyl ersetzt sein kann;
u Null oder 1;
u2 Null oder 1;
v Null oder 1;
wobei der Phenylkern nicht substituiert ist oder substituiert mit 1-3 Substituenten ausgewählt aus der Gruppe bestehend aus F, Cl, CF₃, Methyl, Methoxy, -(CH₂)_{w}N(21)R(22) und NR(18)R(19);
R(18), R(19), R(21) und R(22) unabhängig voneinander H, (C₁-C₄)-Alkyl oder (C₁-C₄)-Perfluoralkyl;
w 1, 2, 3 oder 4;
wobei der Heterocyclus des (C₁-C₉)-Heteroaryl unsubstituiert oder substituiert ist mit 1 - 3 Substituenten ausgewählt aus der Gruppe bestehend aus F, Cl, CF₃, Methyl oder Methoxy;
R(2), R(3), R(4) und R(5) unabhängig voneinander wie R(1) definiert;
oder
R(1) und R(2) oder R(2) und R(3) jeweils gemeinsam -CH-CH=CH-CH-,
das nicht substituiert ist oder substituiert mit 1 - 3 Substituenten ausgewählt aus der Gruppe bestehend aus F, Cl, CF₃, Methyl, Methoxy, -(CH₂)_{w2}NR(24)R(25) und NR(26)R(27);
R(24), R(25), R(26) und R(27) unabhängig voneinander H, (C₁-C₄)-Alkyl oder (C₁-C₄)-Perfluoralkyl;
w2 1, 2, 3 oder 4;
wobei der Rest T im Molekül jedoch nur zweimal vorhanden ist.

4. Verbindungen der Formel I nach einem oder mehreren der Ansprüche 1 bis 3, welche sind:
1,2-Bis-[3-(E-2-methyl-propensäureguanidid)]benzol-dihydrochlorid,
1,3-Bis-[3-(E-2-methyl-propensäureguanidid)]benzol-dihydrochlorid,
1,4-Bis-[3-(E-2-methyl-propensäureguanidid)]benzol-dihydrochlorid,
2,3-Bis-[3-(E-2-methyl-propensäureguanidid)]naphthalin-dihydrochlorid,
1,2-Bis-[3-(Z-2-fluor-propensäureguanidid)]benzol-dihydrochlorid,
1-[3-(Z-2-fluor-propensäureguanidid)]-2-[3-(E-2-methyl-propensäureguanidid)]benzol-dihydrochlorid,
1,3-Bis-[3-(Z-2-fluor-propensäureguanidid)]benzol-dihydrochlorid,
3-(4-Chlor-3-guanidinocarbonyl-5-phenyl)phenyl-2-methyl-propensäureguanidid,
1,3-Bis-[3-(E-2-methyl-propensäureguanidid)]-2-methoxy-5-methyl-benzol-hydrochlorid,
1,2-Bis-[3-(E-2-methyl-propensäureguanidid)]-4-methyl-benzol-dihydrochlorid,
1,2-Bis-[3-(E-2-methyl-propensäureguanidid)]-4,5-dichlor-benzol-dihydrochlorid,
1,3-Bis-[3-(E-propensäureguanidid)]benzol-dihydrochlorid,
1,2-Bis-[3-(E-2-methyl-propensäureguanidid)]-4-brom-benzol-dihydrochlorid,
1,2-Bis-[3-(E-2-methyl-propensäureguanidid)]-4-(4-methoxyphenoxy)-benzol-dihydrochlorid,
1,2-Bis-[3-(E-2-methyl-propensäureguanidid)]-4-(4-methyl-phenoxy)-benzol-dihydrochlorid,
1,3-Bis-[3-(E-2-methyl-propensäureguanidid)]-5-methoxy-benzol-hydrochlorid,
1,3-Bis-[3-(E-2-methyl-propensäureguanidid)]-5-tert.-butyl-benzol-hydrochlorid,
1,4-Bis-[3-(E-2-methyl-propensäureguanidid)]-2,5-dichlor-benzol-dihydrochlorid,
1,2-Bis-[3-(E-2-methyl-propensäureguanidid)]-4-(phenoxy)-ben2ol-dihydrochlorid,
1,2-Bis-[3-(E-2-methyl-propensäureguanidid)]-4-(methoxy)-benzol-dihydroch!orid,
1,2-Bis-[3-(E-2-methyl-propensäureguanidid)]-4-(ethoxy)-benzol-dihydrochlorid und
1,2-Bis-[3-{E-2-methyl-propensäureguanidid)]-4-(3-pyridyloxy)-benzol-dihydrochlorid.

5. Verfahren zur Herstellung einer Verbindung I, dadurch gekennzeichnet, daß man eine Verbindung der Formel II 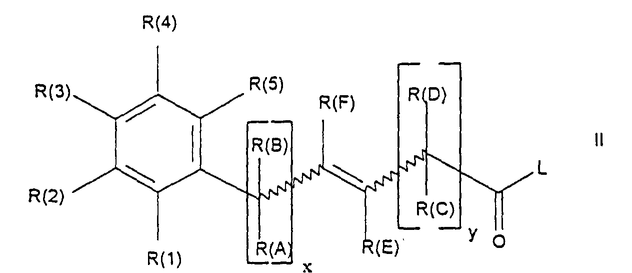 mit Guanidin umsetzt, worin R(1), R(2), R(3), R(4), R(5), R(A), R(B), R(C), R(D), R(E), R(F), x und y die angegebene Bedeutung besitzen und L für eine leichte nucleophil substituierbare Abgangsgruppe steht.

6. Verwendung einer Verbindung I nach Anspruch 1 zur Herstellung eines Medikaments zu Behandlung von Arrhythmien.

7. Verwendung einer Verbindung I nach Anspruch 1 zur Herstellung eines Medikaments zur Behandlung oder Prophylaxe des Herzinfarkts.

8. Verwendung einer Verbindung I nach Anspruch 1 zur Herstellung eines Medikaments zur Behandlung oder Prophylaxe der Angina Pectoris.

9. Verwendung einer Verbindung I nach Anspruch 1 zur Herstellung eines Medikaments zur Behandlung oder Prophylaxe von ischämischen Zuständen des Herzens.

10. Verwendung einer Verbindung I nach Anspruch 1 zur Herstellung eines Medikaments zur Behandlung oder Prophylaxe von ischämischen Zuständen des peripheren und zentralen Nervensystems und des Schlaganfalls.

11. Verwendung einer Verbindung I nach Anspruch 1 zur Herstellung eines Medikaments zur Behandlung oder Prophylaxe von ischämischen Zuständen peripherer Organe und Gliedmaßen.

12. Verwendung einer Verbindung I nach Anspruch 1 zur Herstellung eines Medikaments zur Behandlung von Schockzuständen.

13. Verwendung einer Verbindung I nach Anspruch 1 zur Herstellung eines Medikaments zum Einsatz bei chirurgischen Operationen und Organtransplantationen.

14. Verwendung einer Verbindung I nach Anspruch 1 zur Herstellung eines Medikaments zur Konservierung und Lagerung von Transplantaten für chirurgische Maßnahmen.

15. Verwendung einer Verbindung I nach Anspruch I zur Herstellung eines Medikaments zur Behandlung von Krankheiten, bei denen die Zellproliferation eine primäre oder sekundäre Ursache darstellt.

16. Verwendung einer Verbindung I nach Anspruch 1 zur Herstellung eines Medikaments zur Behandlung oder Prophylaxe von Störungen des Fettstoffwechsels.

17. Verwendung einer Verbindung I nach Anspruch 1 zur Herstellung eines Medikaments zur Behandlung oder Prophylaxe von Störungen von gestörtem Atemantrieb.

18. Verwendung einer Verbindung I nach Anspruch 1 zur Herstellung eines Medikaments zur Behandlung oder zur Prophylaxe von akuten und chronischen Nierenerkrankungen, besonders des akuten Nierenversagens und des chronischen Nierenversagens.

19. Heilmittel, enthaltend eine wirksame Menge einer Verbindung I nach einem oder mehreren der Ansprüche 1 bis 4.

## Claims

1. A phenyl-substituted alkenylcarboxylic acid guanidide of the formula I 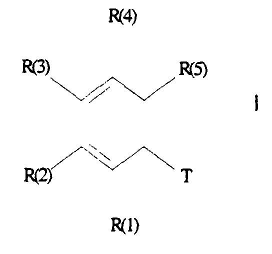 in which:
T is 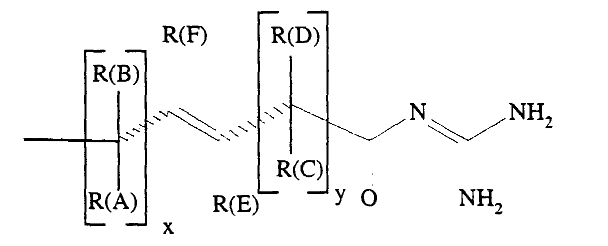
R(A) is hydrogen, F, Cl, Br, I, CN, OH, OR(6), (C₁-C₄)-alkyl, Oᵣ (CH₂) ₐC_{b}F_{2b+1}, (C₃-C₈)-cycloalkyl oder NR(7)R(8)
r is zero or 1;
a is zero, 1, 2, 3 or 4;
b is 1, 2, 3 or 4;
R(6) is (C₁-C₄)-alkyl, (C₁-C₄)-perfluoroalkyl, (C₃-C₆)-alkenyl, (C₃-C₈)-cycloalkyl, phenyl or benzyl,
the phenyl nucleus not being substituted or being substituted by 1 - 3 substituents selected from the group consisting of F, Cl, CF₃, methyl, methoxy and NR(9)R(10);
R(9) and R(10) are H, (C₁-C₄)-alkyl or (C₁-C₄)-perfluoroalkyl;
R(7) and R(8) independently of one another are defined as R(6);
or
R(7) and R(8) together are 4 or 5 methylene groups, of which one CH₂ group can be replaced by oxygen, sulfur, NH, N-CH₃ or N-benzyl;
R(B), R(C) and R(D) independently are defined as R(A);
x is zero, 1 or 2;
y is zero, 1 or 2;
R(F) is hydrogen, F, Cl, Br, I, CN, OR(12), (C₁-C₈)-alkyl, Oₚ(CH₂)_{f}C_{g}F_{2g+1}, (C₃-C₈)-cycloalkyl or (C₁-C₉)-heteroaryl;
p is zero or 1;
f is zero, 1, 2, 3 or 4;
g is 1, 2, 3, 4, 5, 6, 7 or 8;
R(12) is (C₁-C₈)-alkyl, (C₁-C₄) -perfluoroalkyl, (C₃-C₈)-alkenyl, (C₃-C₈)-cyclo-alkyl, phenyl or benzyl
the phenyl nucleus not being substituted or being substituted by 1-3 substituents selected from the group consisting of F, Cl, CF₃, methyl, methoxy and NR(13)R(14);
R(13) and R(14) are H, (C₁-C₄)-alkyl or (C₁-C₄)-perfluoroalkyl;
R(E) is defined as R(F);
R(1) is defined as T;
or
R(1) is hydrogen, -OₖCₘH₂ₘ₊₁, -Oₙ(CH₂)ₚC_{q}F_{2q+1}, F, Cl, Br, I, CN, -(C=O)-N=C(NH₂)₂, -SOᵣR(17), SOᵣ₂NR(31)R(32), -Oᵤ(CH₂)ᵥC₆H₅, -Oᵤ₂- (C₁-C₉) -heteroaryl or -Sᵤ₂- (C₁-C₉)-heteroaryl;
k is zero or 1;
m is zero, 1, 2, 3, 4, 5, 6, 7 or 8;
n is zero or 1;
p is zero, 1, 2, 3 or 4;
q is 1, 2, 3, 4, 5, 6, 7 or 8;
r is zero, 1, 2;
r2 is zero, 1, 2;
R(31) and R(32) independently of one another are hydrogen, (C₁--C₈)-alkyl or (C₁-C₈)-perfluoroalkyl;
or
R(31) and R(32) together are 4 or 5 methylene groups, of which one CH₂ group can be replaced by oxygen, S, NH, N-CH₃ or N-benzyl;
R(17) is (C₁-C₈)-alkyl;
u is zero or 1;
u2 is zero or 1;
v is zero, 1, 2, 3 or 4;
the phenyl nucleus not being substituted or being substituted by 1 - 3 substituents selected from the group consisting of F, Cl, CF₃, methyl, methoxy, -(CH₂)_{w}NR(21)R(22), NR(18)R(19) and (C₁-C₉)-heteroaryl;
R(18), R(19), R(21) and R(22) independently of one another are (C₁-C₄)-alkyl or (C₁-C₄)-perfluoroalkyl;
w is 1, 2, 3 or 4;
the heterocycle of the (C₁-C₉)-heteroaryl being unsubstituted or substituted by 1 - 3 substituents selected from the group consisting of F, Cl, CF₃, methyl or methoxy;
R(2), R(3), R(4) and R(5) independently of one another are defined as R(1),
or
R(1) and R(2) or R(2) and R(3) in each case together are -CH-CH=CH-CH-,
which is not substituted or is substituted by 1 - 3 substituents selected from the group consisting of F, Cl, CF₃, methyl, methoxy,-(CH₂)_{w2}NR(24)R(25) and NR(26)R(27);
R(24), R(25), R(26) and R(27) are H, (C₁-C₄)-alkyl or (C₁-C₄)perfluoroalkyl;
w2 is 1, 2, 3 or 4;
the radical T being present in the molecule at least twice, but only three times at most;
or its pharmaceutically tolerable salts.

2. A compound of the formula I as claimed in claim 1, in which
T is 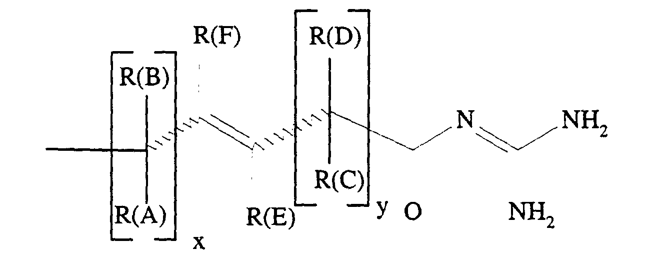
R(A) is hydrogen, F, Cl, CN, OH, OR(6), (C₁-C₄)-alkyl, Oᵣ(CH₂)ₐC_{b}F_{2b+1}, (C₃-C₈)-cycloalkyl or NR(7)R(8)
r is zero or 1;
a is zero, 1 or 2;
b is 1, 2, 3 or 4;
R(6) is (C₁-C₄)-alkyl, (C₁-C₄)-perfluoroalkyl, phenyl or benzyl,
the phenyl nucleus not being substituted or being substituted by 1 - 3 substituents selected from the group consisting of F, Cl, CF₃, methyl, methoxy and NR(9)R(10);
R(9) and R(10) independently of one another are H, CH₃ or CF₃;
R(7) and R(8) independently of one another are defined as R(6);
or
R(7) and R(8) together are 4 or 5 methylene groups, of which one CH₂ group can be replaced by oxygen, sulfur, NH, N-CH₃ or N-benzyl;
R(B), R(C), R(D) independently are defined as R(A);
x is zero or 1;
y is zero or 1;
R(F) is hydrogen, F, Cl, CN, OR(12), (C₁-C₄)-alkyl, Oₚ(CH₂)_{f}C_{g}F_{2g+1}, (C₃-C₈)-cycloalkyl or (C₁-C₉)-heteroaryl;
p is zero or 1;
f is zero, 1 or 2;
g is 1, 2, 3 or 4;
R(l2) is (C₁-C₄)-alkyl, (C₁-C₄)-perfluoroalkyl, (C₃-C₈)-cycloalkyl, phenyl or benzyl,
the phenyl nucleus not being substituted or being substituted by 1 - 3 substituents selected from the group consisting of F, Cl, CF₃, methyl, methoxy and NR(13)R(14);
R(13) and R(14) independently of one another are H, CH₃ or CF₃;
R(E) is defined as R(F);
R(1) is defined as T;
or
R(1) is hydrogen, -OₖCₘH₂ₘ₊₁, -OₙC_{q}F_{2q+1}, F, Cl, Br, I, CN,
- (C=O)-N=C(NH₂)₂, -SOᵣR(17), -SOᵣ₂NR(31)R(32 ), -Oᵤ(CH₂)ᵥC₆H₅, -Oᵤ₂- (C₁-C₉)-heteroaryl or -Sᵤ₂-(C₁-C₉)-heteroaryl;
k is zero or 1;
m is zero, 1, 2, 3 or 4;
n is zero or 1;
q is 1, 2, 3 or 4;
r is 2;
r2 is zero, 1, 2;
R(31) and R(32) independently of one another are hydrogen, (C₁-C₄)-alkyl or (C₁-C₄) -perfluoroalkyl;
or
R(31) and R(32) together are 4 or 5 methylene groups, of which one CH₂ group can be replaced by oxygen, S, NH, N-CH₃ or N-benzyl;
R(17) is (C₁-C₄)-alkyl;
u is zero or 1;
u2 is zero or 1;
v is zero, 1 or 2;
the phenyl nucleus not being substituted or being substituted by 1 - 3 substituents selected from the group consisting of F, Cl, CF₃, methyl, methoxy, -(CH₂)_{w}N(21)R(22), NR(18)R(19) or (C₁-C₉)-heteroaryl;
R(18), R(19), R(21) and R(22) are H, (C₁-C₄)-alkyl or (C₁-C₄)-perfluoroalkyl;
w is 1, 2, 3, 4;
the heterocycle of the (C₁-C₉)-heteroaryl being unsubstituted or substituted by 1 - 3 substituents selected from the group consisting of F, Cl, CF₃, methyl or methoxy;
R(2), R(3), R(4) and R(5) independently of one another are defined as R(1),
or
R(1) and R(2) or R(2) and R(3) in each case together are -CH-CH=CH-CH-,
which is not substituted or is substituted by 1 - 3 substituents selected from the group consisting of F, Cl, CF₃, methyl, methoxy,-(CH₂)_{w2}NR(24)R(25) and NR(26)R(27);
R(24), R(25), R(26) and R(27) are H, CH3 or CF₃;
w2 is 1, 2, 3 or 4;
the radical T only being present twice, however, in the molecule.

3. A compound of the formula I as claimed in claim 1 or 2, in which:
T is 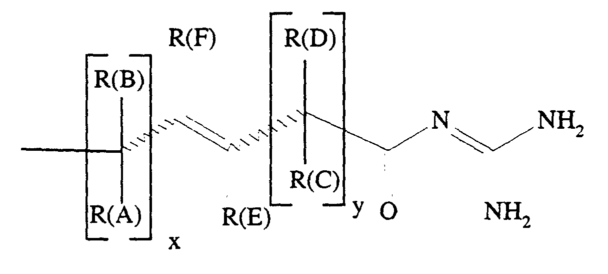
x is zero;
y is zero;
R(F) is hydrogen, F, Cl, CN, OR(12), (C₁-C₄)-alkyl, -OₚC_{g}F_{2g+1}, (C₃-C₈)-cycloalkyl or (C₁-C₉)-heteroaryl;
p is zero or 1;
g is 1, 2, 3 or 4;
R(12) is (C₁-C₄)-alkyl, CF₃, (C₃-C₈)-cycloalkyl, phenyl or benzyl,
the phenyl nucleus in each case not being substituted or being substituted by 1 - 3 substituents selected from the group consisting of F, Cl, CF₃, methyl, methoxy and NR(13)R(14);
R(13) and R(14) are H, CH₃ or CF₃;
R(E) is defined as R(F);
R(1) is defined as T;
or
R(1) is hydrogen, -OₖCₘH₂ₘ₊₁, -OₙC_{q}F_{2q+1}, F, Cl, CN, -(C=O)-N=C(NH₂)₂, -SO₂CH₃, -SO₂NR(31)R(32), -Oᵤ(CH₂)ᵥC₆H₅, -Oᵤ₂- (C₁-C₉)-heteroaryl or -Sᵤ₂-(C₁-C₉)-heteroaryl;
k is zero or 1;
m is zero, 1, 2, 3 or 4;
n is zero or 1;
q is 1, 2, 3 or 4;
R(31) and R(32) independently of one another are hydrogen or (C₁-C₄)-alkyl;
or
R(31) and R(32) together are 4 or 5 methylene groups, of which one CH₂ group can be replaced by oxygen, S, NH, N-CH₃ or N-benzyl;
u is zero or 1;
u2 is zero or 1;
v is zero or 1;
the phenyl nucleus not being substituted or being substituted by 1 - 3 substituents selected from the group consisting of F, Cl, CF₃, methyl, methoxy, -(CH₂)_{w}N(21)R(22) and NR(18)R(19);
R(18), R(19), R(21) and R(22) independently of one another are H, (C₁-C₄)-alkyl or (C₁-C₄)-perfluoroalkyl;
w is 1, 2, 3 or 4;
the heterocycle of the (C₁-C₉)-heteroaryl being unsubstituted or substituted by 1 - 3 substituents selected from the group consisting of F, Cl, CF₃, methyl or methoxy;
R(2), R(3), R(4) and R(5) independently of one another are defined as R(1);
or
R(1) and R(2) or R(2) and R(3) in each case together are -CH-CH=CH-CH-,
which is not substituted or is substituted by 1 - 3 substituents selected from the group consisting of F, Cl, CF₃, methyl, methoxy,-(CH₂)_{w2}NR(24)R(25) and NR(26)R(27);
R(24), R(25), R(26) and R(27) independently of one another are H, (C₁-C₄)-alkyl or (C₁-C₄)-perfluoroalkyl;
w2 is 1, 2, 3 or 4;
the radical T only being present twice, however, in the molecule.

4. A compound of the formula I as claimed in one or more of claims 1 to 3, which is:
1,2-Bis[3-(E-2-methylpropenoic acid guanidide)]benzene dihydrochloride,
1,3-Bis[3-(E-2-methylpropenoic acid guanidide)]benzene dihydrochloride,
1,4-Bis[3-(E-2-methylpropenoic acid guanidide)]benzene dihydrochloride,
2,3-Bis[3-(E-2-methylpropenoic acid guanidide)]naphthalene dihydrochloride,
1,2-Bis[3-(Z-2-fluoropropenoic acid guanidide)]benzene dihydrochloride,
1-[3-(Z-2-fluoropropenoic acid guanidide)]-2-[3-(E-2-methylpropenoic acid guanidide)]benzene dihydrochloride,
1,3-Bis[3-(Z-2-fluoropropenoic acid guanidide)]benzene dihydrochloride,
3-(4-chloro-3-guanidinocarbonyl-5-phenyl)phenyl-2-methylpropenoic acid guanidide,
1,3-Bis[3-(E-2-methylpropenoic acid guanidide)]-2-methoxy-5-methyl-benzene hydrochloride,
1,2-Bis[3-(E-2-methylpropenoic acid guanidide)]-4-methylbenzene dihydrochloride,
1,2-Bis[3-(E-2-methylpropenoic acid guanidide)]-4,5-dichlorobenzene dihydrochloride,
1,3-Bis[3-(E-propenoic acid guanidide)]benzene dihydrochloride,
1,2-Bis[3-(E-2-methylpropenoic acid guanidide)]-4-bromobenzene dihydrochloride,
1,2-Bis[3-(E-2-methylpropenoic acid guanidide)]-4-(4-methoxyphenoxy)-benzene dihydrochloride,
1,2-Bis[3-(E-2-methylpropenoic acid guanidide)]-4-(4-methylphenoxy)-benzene dihydrochloride,
1,3-Bis[3-(E-2-methylpropenoic acid guanidide)]-5-methoxybenzene hydrochloride,
1,3-Bis[3-(E-2-methylpropenoic acid guanidide)]-5-tert-butylbenzene hydrochloride,
1;4-Bis[3-(E-2-methylpropenoic acid guanidide)]-2,5-dichlorobenzene dihydrochloride,
1,2-Bis[3-(E-2-methylpropenoic acid guanidide)]-4-(phenoxy)benzene dihydrochloride,
1,2-Bis[3-(E-2-methylpropenoic acid guanidide)]-4-(methoxy)benzene dihydrochloride,
1,2-Bis[3-(E-2-methylpropenoic acid guanidide)]-4-(ethoxy)benzene dihydrochloride and
1,2-Bis[3-(E-2-methylpropenoic acid guanidide)]-4-(3-pyridyloxy)benzene dihydrochloride.

5. A process for the preparation of a compound I, which comprises reacting a compound of the formula II 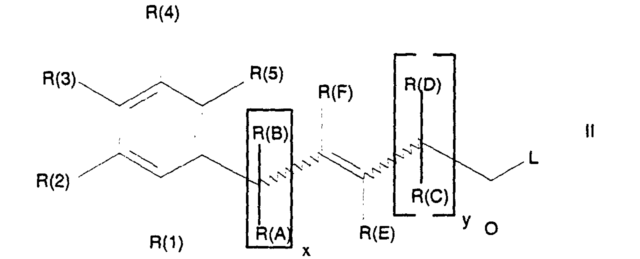 in which R(1), R(2), R(3), R(4), R(5), R(A), R(B), R(C), R(D), R(E), R(F), x and y have the meaning indicated and L is an easily nucleophilically substitutable leaving group, with guanidine.

6. The use of a compound I as claimed in claim 1 for the production of a medicament for treatment of arrhythmias.

7. The use of a compound I as claimed in claim 1 for the production of a medicament for the treatment or prophylaxis of cardiac infarct.

8. The use of a compound I as claimed in claim 1 for the production of a medicament for the treatment or prophylaxis of angina pectoris.

9. The use of a compound I as claimed in claim 1 for the production of a medicament for the treatment or prophylaxis of ischemic conditions of the heart.

10. The use of a compound I as claimed in claim 1 for the production of a medicament for the treatment or prophylaxis of ischemic conditions of the peripheral and central nervous system and of stroke.

11. The use of a compound I as claimed in claim 1 for the production of a medicament for the treatment or prophylaxis of ischemic conditions of peripheral organs and members.

12. The use of a compound I as claimed in claim 1 for the production of a medicament for the treatment of states of shock.

13. The use of a compound I as claimed in claim 1 for the production of a medicament for use in surgical operations and organ transplantation.

14. The use of a compound I as claimed in claim 1 for the production of a medicament for the preservation and storage of transplants for surgical measures.

15. The use of a compound I as claimed in claim 1 for the production of a medicament for the treatment of illnesses in which cell proliferation is a primary or secondary cause.

16. The use of a compound I as claimed in claim 1 for the production of a medicament for the treatment or prophylaxis of disorders of lipid metabolism.

17. The use of a compound I as claimed in claim 1 for the production of a medicament for the treatment or prophylaxis of impaired respiratory drive.

18. The use of a compound I as claimed in claim 1 for the production of a medicament for the treatment or prophylaxis of acute and chronic kidney diseases, particularly of acute kidney failure and of chronic kidney failure.

19. A therapeutic comprising an effective amount of a compound I as claimed in one or more of claims 1 to 4.

## Revendications

1. Alcénoylguanidides substitués par un groupe phényle, de formule I 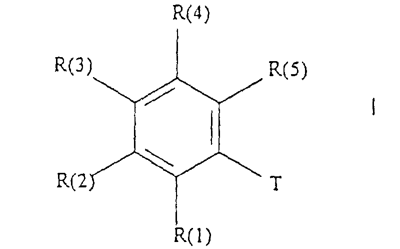 dans laquelle
T représente 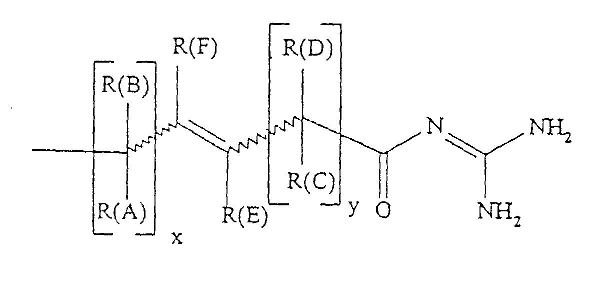
R(A) représente un atome d'hydrogène ou de F, Cl, Br, I, ou un groupe CN, OH, OR(6), alkyle en C₁-C₄, Oᵣ(CH₂)ₐC_{b}F_{2b+1}, cycloalkyle en C₃-C₈ ou NR(7)R(8);
r est zéro ou 1;
a est zéro, 1, 2, 3 ou 4;
b est 1, 2, 3 ou 4;
R(6) représente un groupe alkyle en C₁-C₄, perfluoroalkyle en C₁-C₄, alcényle en C₃-C₆, cycloalkyle en C₃-C₈, phényle ou benzyle,
le noyau phényle n'étant pas substitué ou portant 1-3 substituants choisis dans l'ensemble constitué par les atomes de F, Cl et les groupes CF₃, méthyle, méthoxy et NR(9)R(10),
R(9) et R(10) représentant H ou un groupe alkyle en C₁-C₄ ou perfluoroalkyle en C₁-C₄;
R(7) et R(8) indépendamment l'un de l'autre, sont définis comme R(6),
ou
R(7) et R(8) représentent ensemble 4 ou 5 groupes méthylène, dont un groupe CH₂ peut être remplacé par un atome d'oxygène, de soufre ou par un groupe NH, N-CH₃ ou N-benzyle;
R(B), R(C) et R(D) sont indépendamment définis comme R(A) ;
x est égal à zéro, 1 ou 2 ;
y est égal à zéro, 1 ou 2 ;
R(F) représente un atome d'hydrogène, de F, Cl, Br, I, ou un groupe CN, OR(12), alkyle en C₁-C₈, Oₚ(CH₂)_{f}C_{g}F_{2g+1}, cycloalkyle en C₃-C₈ ou hétéroaryle en C₁-C₉,
p est zéro ou 1,
f est zéro, 1, 2, 3 ou 4,
g est 1, 2, 3, 4, 5, 6, 7 ou 8,
R(12) représente un groupe alkyle en C₁-C₈, perfluoroalkyle en C₁-C₄, alcényle en C₃-C₈, cycloalkyle en C₃-C₈, phényle ou benzyle;
le noyau phényle n'étant pas substitué ou portant 1-3 substituants choisis dans l'ensemble constitué par les atomes de F, Cl et les groupes CF₃, méthyle, méthoxy et NR(13)R(14),
R(13) et R(14) représentant H ou un groupe alkyle en C₁C₄ ou perfluoroalkyle en C₁-C₄;
R(E) est, indépendamment, défini comme R(F) ;
R(1) est indépendamment défini comme T;
ou
R(1) représente un atome d'hydrogène ou un groupe -OₖCₘH₂ₘ₊₁,
-Oₙ(CH₂)ₚC_{q}F_{2q+1}, F, Cl, Br, I, un groupe CN, -(C=O)-N=C(NH₂)₂, -SOᵣR(17), -SOᵣ₂NR(31)R(32), -Oᵤ(CH₂)ᵥC₆H₅, -Oᵤ₂-hétéroaryle (C₁-C₉) ou -Sᵤ₂-hétéroaryle (C₁-C₉);
k est zéro ou 1;
m est zéro, 1, 2, 3, 4, 5, 6, 7 ou 8;
n est zéro ou 1;
p est zéro, 1, 2, 3 ou 4;
q est 1, 2, 3, 4, 5, 6, 7 ou 8;
r est zéro, 1 ou 2;
r2 est zéro, 1 ou 2;
R(31) et R(32) représentent, indépendamment l'un de l'autre, un atome d'hydrogène ou un groupe alkyle en C₁-C₈ ou perfluoroalyle en C₁-C₈;
ou
R(31) et R(32) forment ensemble 4 ou 5 groupes méthylène, dont un groupe CH₂ peut être remplacé par un atome d'oxygène ou de soufre ou par un groupe NH, N-CH₃ ou N-benzyle;
R(17) représente un groupe alkyle en C₁-C₈;
u est zéro ou 1;
u2 est zéro ou 1;
v est zéro, 1, 2, 3 ou 4;
le noyau phényle n'étant pas substitué ou portant 1-3 substituants choisis dans l'ensemble constitué par les atomes de F, Cl et les groupes CF₃, méthyle, méthoxy, -(CH₂)_{w}NR(21)R(22), NR(18)R(19) et hétéroaryle en C₁-C₉,
R(18), R(19), R(21) et R(22) représentant indépendamment les uns des autres un groupe alkyle en C₁-C₄ ou perfluoroalkyle en C₁-C₄;
w est 1, 2, 3 ou 4;
l'hétérocycle du groupe hétéroaryle en C₁-C₉ n'étant pas substitué ou portant 1-3 substituants choisis dans l'ensemble constitué par les atomes de F, Cl et les groupes CF₃, méthyle et méthoxy;
R(2), R(3), R(4) et R(5) sont, indépendamment les uns des autres, définis comme R(1),
ou
R(1) et R(2) ou R(2) et R(3) forment ensemble, respectivement, un groupe -CH-CH=CH-CH-
qui n'est pas substitué ou porte 1-3 substituants choisis dans l'ensemble constitué par les atomes de F, Cl et les groupes CF₃, méthyle, méthoxy, -(CH₂)_{w2}NR(24)R(25) et NR(26)R(27);
R(24), R(25), R(26) et R(27) représentant H ou un groupe alkyle en C₁-C₄ ou perfluoroalkyle en C₁-C₄;
w2 est 1, 2, 3 ou 4;
le radical T étant présent au moins deux fois, mais seulement trois fois au maximum dans la molécule ;
ainsi que leurs sels pharmaceutiquement acceptables.

2. Composé de formule I selon la revendication 1, dans lequel:
T représente 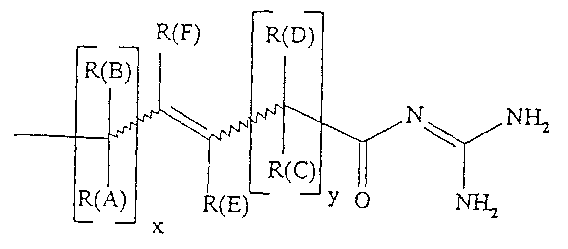
R(A) représente un atome d'hydrogène ou de F, Cl, ou un groupe CN, OH, OR(6), alkyle en C₁-C₄, Oᵣ(CH₂)ₐC_{b}F_{2b+1}, cycloalkyle en C₃-C₈ ou NR(7)R(8);
r est zéro ou 1;
a est zéro, 1 ou 2;
b est 1, 2, 3 ou 4;
R(6) représente un groupe alkyle en C₁-C₄, perfluoroalkyle en C₁-C₄, phényle ou benzyle,
le noyau phényle n'étant pas substitué ou portant 1-3 substituants choisis dans l'ensemble constitué par les atomes de F, Cl et les groupes CF₃, méthyle, méthoxy et NR(9)R(10),
R(9) et R(10) représentant, indépendamment l'un de l'autre, H, CH₃ ou CF₃;
R(7) et R(8) indépendamment l'un de l'autre, sont définis comme R(6),
ou
R(7) et R(8) représentent ensemble 4 ou 5 groupes méthylène, dont un groupe CH₂ peut être remplacé par un atome d'oxygène, de soufre ou par un groupe NH, N-CH₃ ou N-benzyle;
R(B), R(C), R(D) sont indépendamment définis comme R(A);
x est égal à zéro ou 1;
y est égal à zéro ou 1;
R(F) représente un atome d'hydrogène, de F, Cl, ou un groupe CN, OR(12), alkyle en C₁-C₄, Oₚ(CH₂)_{f}C_{g}F_{2g+1}, cycloalkyle en C₃-C₈ ou hétéroaryle en C₁-C₉,
p est zéro ou 1,
f est zéro, 1 ou 2,
g est 1, 2, 3 ou 4,
R(12) représente un groupe alkyle en C₁-C₄, perfluoroalkyle en C₁-C₄, cycloalkyle en C₃-C₈, phényle ou benzyle;
le noyau phényle n'étant pas substitué ou portant 1-3 substituants choisis dans l'ensemble constitué par les atomes de F, Cl et les groupes CF₃, méthyle, méthoxy et NR(13)R(14),
R(13) et R(14) représentant, indépendamment l'un de l'autre, H, CH₃ ou CF₃;
R(E) est, indépendamment, défini comme R(F);
R(1) est indépendamment défini comme T;
ou
R(1) représente un atome d'hydrogène ou un groupe-OₖCₘH₂ₘ₊₁,
-OₙC_{q}F_{2q+1}, F, Cl, Br, I, un groupe CN, -(C=O)-N=C(NH₂)₂, -SOᵣR(17), -SOᵣ₂NR(31)R(32), -Oᵤ(CH₂)ᵥC₆H₅, -Oᵤ₂-hétéroaryle(C₁-C₉) ou -Sᵤ₂-hétéroaryle (C₁-C₉);
k est zéro ou 1;
m est zéro, 1, 2, 3 ou 4;
n est zéro ou 1;
q est 1, 2, 3 ou 4;
r est égal à 2;
r2 est zéro, 1 ou 2;
R(31) et R(32) représentent, indépendamment l'un de l'autre, un atome d'hydrogène ou un groupe alkyle en C₁-C₄ ou perfluoroalyle en C₁-C₄;
ou
R(31) et R(32) forment ensemble 4 ou 5 groupes méthylène, dont un groupe CH₂ peut être remplacé par un atome d'oxygène ou de soufre ou par un groupe NH, N-CH₃ ou N-benzyle;
R(17) représente un groupe alkyle en C₁-C₄;
u est zéro ou 1;
u2 est zéro ou 1;
v est zéro, 1 ou 2 ;
le noyau phényle n'étant pas substitué ou portant 1-3 substituants choisis dans l'ensemble constitué par les atomes de F, Cl et les groupes CF₃, méthyle, méthoxy, -(CH₂)_{w}NR(21)R(22), NR(18)R(19) et hétéroaryle en C₁-C₉,
R(18), R(19), R(21) et R(22) représentant H ou un groupe alkyle en C₁-C₄ ou perfluoroalkyl en C₁-C₄;
w est 1, 2, 3 ou 4;
l'hétérocycle du groupe hétéroaryle en C₁-C₉ n'étant pas substitué ou portant 1-3 substituants choisis dans l'ensemble constitué par les atomes de F, Cl et les groupes CF₃, méthyle et méthoxy;
R(2), R(3), R(4) et R(5) sont, indépendamment les uns des autres, définis comme R(1),
ou
R(1) et R(2) ou R(2) et R(3) forment ensemble, respectivement, un groupe -CH-CH=CH-CH-
qui n'est pas substitué ou porte 1-3 substituants choisis dans l'ensemble constitué par les atomes de F, Cl et les groupes CF₃, méthyle, méthoxy, -(CH₂)_{w2}NR(24)R(25) et NR(26)R(27);
R(24), R(25), R(26) et R(27) représentant H, CH₃ ou CF₃;
w2 est 1, 2, 3 ou 4;
le radical T n'étant toutefois présent que deux fois dans la molécule.

3. Composé de formule I selon la revendication 1 ou 2, dans lequel:
T représente 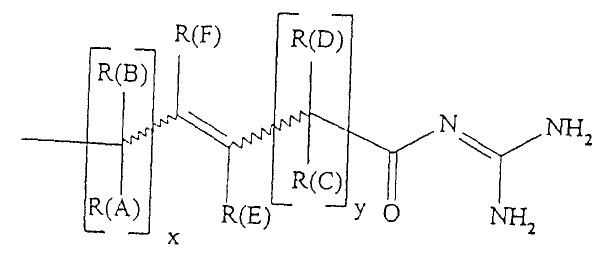
x est égal à zéro ;
y est égal à zéro ;
R(F) représente un atome d'hydrogène, de F, Cl, ou un groupe CN, OR(12), alkyle en C₁-C₄, -OₚC_{g}F_{2g+1}, cycloalkyle en C₃-C₈ ou hétéroaryle en C₁-C₉,
p est zéro ou 1,
g est 1, 2, 3 ou 4,
R(12) représente un groupe alkyle en C₁-C₄, CF₃, cycloalkyle en C₃-C₈, phényle ou benzyle;
le noyau phényle dans chaque cas n'étant pas substitué ou portant 1-3 substituants choisis dans l'ensemble constitué par les atomes de F, Cl et les groupes CF₃, méthyle, méthoxy et NR(13)R(14),
R(13) et R(14) représentant H, CH₃ ou CF₃;
R(E) est, indépendamment, défini comme R(F);
R(1) est indépendamment défini comme T;
ou
R(1) représente un atome d'hydrogène ou un groupe -OₖCₘH₂ₘ₊₁, -OₙC_{q}F_{2q+1}, F, Cl, un groupe CN, -(C=O)-N=C(NH₂)₂, -SO₂CH₃, -SO₂NR(31)R(32), -Oᵤ(CH₂)ᵥC₆H₅, -Oᵤ₂-hétéroaryle (C₁-C₉) ou -Sᵤ₂-hétéroaryle (C₁-C₉);
k est zéro ou 1;
m est zéro, 1, 2, 3 ou 4;
n est zéro ou 1;
q est 1, 2, 3 ou 4 ;
R(31) et R(32) représentent, indépendamment l'un de l'autre, un atome d'hydrogène ou un groupe alkyle en C₁-C₄;
ou
R(31) et R(32) forment ensemble 4 ou 5 groupes méthylène, dont un groupe CH₂ peut être remplacé par un atome d'oxygène ou de soufre ou par un groupe NH, N-CH₃ ou N-benzyle;
u est zéro ou 1;
u2 est zéro ou 1;
v est zéro ou 1;
le noyau phényle n'étant pas substitué ou portant 1-3 substituants choisis dans l'ensemble constitué par les atomes de F, Cl et les groupes CF₃, méthyle, méthoxy, -(CH₂)_{w}NR(21)R(22) et NR(18)R(19),
R(18), R(19), R(21) et R(22) représentant, indépendamment les uns des autres, H ou un groupe alkyle en C₁-C₄ ou perfluoroalkyle en C₁-C₄;
w est 1, 2, 3 ou 4;
l'hétérocycle du groupe hétéroaryle en C₁-C₉ n'étant pas substitué ou portant 1-3 substituants choisis dans l'ensemble constitué par les atomes de F, Cl et les groupes CF₃, méthyle et méthoxy;
R(2), R(3), R(4) et R(5) sont, indépendamment les uns des autres, définis comme R(1),
ou
R(1) et R(2) ou R(2) et R(3) forment ensemble, respectivement, un groupe -CH-CH=CH-CH- qui n'est pas substitué ou porte 1-3 substituants choisis dans l'ensemble constitué par les atomes de F, Cl et les groupes CF₃, méthyle, méthoxy, -(CH₂)_{w2}NR(24)R(25) et NR(26)R(27);
R(24), R(25), R(26) et R(27) représentant, indépendamment les uns des autres, H ou un groupe alkyle en C₁-C₄ ou perfluoroalkyle en C₁-C₄;
w2 est 1, 2, 3 ou 4;
le radical T n'étant toutefois présent que deux fois dans la molécule.

4. Composés de formule I selon une ou plusieurs des revendications 1 à 3, qui sont:
le 1,2-bis(3-(E-2-méthyl-propénoylguanidide)]benzène dichlorhydrate,
le 1,3-bis[3-(E-2-méthyl-propénoylguanidide)]benzène dichlorhydrate,
le 1,4-bis[3-(E-2-méthyl-propénoylguanidide)benzène dichlorhydrate,
le 2,3-bis[3-(E-2-méthyl-propénoylguanidide)]naphtalène dichlorhydrate,
le 1,2-bis[3-(Z-2-fluoro-propénoylguanidide)]benzène dichlorhydrate,
le 1-[3-(Z-2-fluoro-propénoylguanidide)]-2-[3-(E-2-méthyl-propénoylguanidide)]benzène dichlorhydrate,
le 1,3-bis[3-(Z-2-fluoro-propénoylguanidide)]benzène dichlorhydrate,
le 3-(4-chloro-3-guanidinocarbonyl-5-phényl)phényl-2-méthylpropénoylguanidide,
le 1,3-bis[3-(E-2-méthyl-propénoylguanidide)]-2-méthoxy-5-méthyl-benzène chlorhydrate,
le 1,2-bis[3-(E-2-méthyl-propénoylguanidide)]-4-méthyl-benzène dichlorhydrate,
le 1,2-bis[3-(E-2-méthyl-propénoylguanidide)]-4,5-dichlorobenzène dichlorhydrate,
le 1,3-bis[3-(E-propénoylguanidide)]benzène dichlorhydrate,
le 1,2-bis[3-(E-2-méthyl-propénoylguanidide)]-4-bromobenzène dichlorhydrate,
le 1,2-bis[3-(E-2-méthyl-propénoylguanidide)]-4-(4-méthoxyphénoxy)-benzène dichlorhydrate,
le 1,2-bis[3-(E-2-méthyl-propénoylguanidide)]-4-(4-méthylphénoxy)-benzène dichlorhydrate,
le 1,3-bis[3-(E-2-méthyl-propénoylguanidide)]-5-méthoxybenzène chlorhydrate,
le 1,3-bis[3-(E-2-méthyl-propénoylguanidide)]-5-tert-butylbenzène chlorhydrate,
le 1,4-bis[3-(E-2-méthyl-propénoylguanidide)]-2,5-dichlorobenzène dichlorhydrate,
le 1,2-bis[3-(E-2-méthyl-propénoylguanidide)]-4-(phénoxy)benzène dichlorhydrate,
le 1,2-bis[3-(E-2-méthyl-propénoylguanidide)]-4-(méthoxy)benzène dichlorhydrate,
le 1,2-bis[3-(E-2-méthyl-propénoylguanidide)]-4-(éthoxy)benzène dichlorhydrate et
le 1,2-bis[3-(E-2-méthyl-propénoylguanidide)]-4-(3-pyridyloxy)benzène dichlorhydrate.

5. Procédé pour la préparation d'un composé I, caractérisé en ce que l'on fait réagir avec de la guanidine un composé de formule II 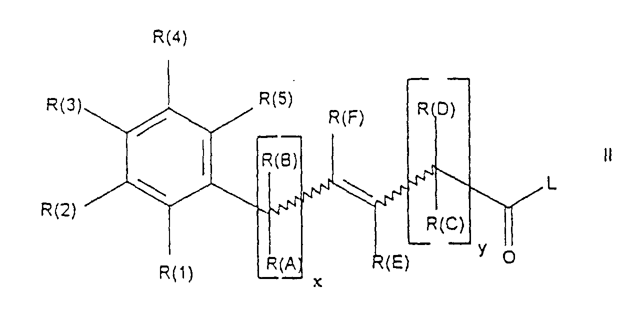 dans laquelle R(1), R(2), R(3), R(4), R(5), R(A), R(B), R(C), R(D), R(E), R(F), x et y ont les significations indiquées et L représente un groupe partant pouvant être aisément remplacé par substitution nucléophile.

6. Utilisation d'un composé I selon la revendication 1, pour la fabrication d'un médicament destiné au traitement d'arythmies.

7. Utilisation d'un composé I selon la revendication 1, pour la fabrication d'un médicament destiné au traitement ou à la prophylaxie de l'infarctus du myocarde.

8. Utilisation d'un composé I selon la revendication 1, pour la fabrication d'un médicament destiné au traitement ou à la prophylaxie de l'angine de poitrine.

9. Utilisation d'un composé I selon la revendication 1, pour la fabrication d'un médicament destiné au traitement ou à la prophylaxie d'états ischémiques du coeur.

10. Utilisation d'un composé I selon la revendication 1, pour la fabrication d'un médicament destiné au traitement ou à la prophylaxie d'états ischémiques du système nerveux périphérique et central et de l'accident cérébral.

11. Utilisation d'un composé I selon la revendication 1, pour la fabrication d'un médicament destiné au traitement ou à la prophylaxie d'états ischémiques d'organes périphériques et des membres.

12. Utilisation d'un composé I selon la revendication 1, pour la fabrication d'un médicament destiné au traitement d'états de choc.

13. Utilisation d'un composé I selon la revendication 1, pour la fabrication d'un médicament destiné à l'utilisation dans des opérations chirurgicales et des transplantations d'organes.

14. Utilisation d'un composé I selon la revendication 1, pour la fabrication d'un médicament destiné à la conservation et au stockage de transplants pour des opérations chirurgicales.

15. Utilisation d'un composé I selon la revendication 1, pour la fabrication d'un médicament destiné au traitement de maladies dans lesquelles la prolifération cellulaire représente une cause primaire ou secondaire.

16. Utilisation d'un composé I selon la revendication 1, pour la fabrication d'un médicament destiné au traitement ou à la prophylaxie de troubles du métabolisme.

17. Utilisation d'un composé I selon la revendication 1, pour la fabrication d'un médicament destiné au traitement ou à la prophylaxie de troubles de l'impulsion respiratoire perturbée.

18. Utilisation d'un composé I selon la revendication 1, pour la fabrication d'un médicament destiné au traitement ou à la prophylaxie d'affections rénales aiguës ou chroniques, en particulier de l'insuffisance rénale aiguë et de l'insuffisance rénale chronique.

19. Médicament, caractérisé en ce qu'il contient une quantité efficace d'un composé I selon une ou plusieurs des revendications 1 à 4.
